# EUROPEAN PATENT APPLICATION

(11) **EP 3 786 973 A1**
(43) Date of publication of application: **03.03.2021**
(21) Application number: 19793974.7
(22) Date of filing: 24.04.2019
(51) Int. Cl.: G16H 40/20, G06Q 50/22

(54) **ENDOSCOPIC EXAMINATION INFORMATION ANALYSIS DEVICE, ENDOSCOPIC EXAMINATION INFORMATION INPUT SUPPORTING SYSTEM, AND ENDOSCOPIC EXAMINATION INFORMATION ANALYSIS METHOD**

(30) Priority: 27.04.2018 JP 2018086190
(71) Applicant: FUJIFILM CORPORATION, Minato-ku Tokyo 106-8620 (JP)
(72) Inventor: OHASHI, Yosuke, Ashigarakami-gun, Kanagawa 258-8538 (JP)
(74) Representative: Klunker IP Patentanwälte PartG mbB
(86) International application number: PCT/JP2019/017487
(87) International publication number: WO 2019/208654

(57) **Abstract**

An object of the present invention is to provide an endoscopic examination information analysis apparatus and an endoscopic examination information analysis method capable of enabling a user to easily grasp a use status of an examination use article and an execution status of a procedure, and an endoscopic examination information input support system capable of efficiently supporting an input of examination information. In accordance with an endoscopic examination information analysis apparatus according to a first aspect of the present invention, since an examination database is updated based on examination information generated whenever an examination is executed, use information of an examination use article is calculated based on a use history of the examination use article for a selected examination item, execution information of a procedure is calculated based on execution history of the procedure, and the calculated use information and execution information are displayed on a display device for each examination use article or procedure, a user can easily grasp the use status of the examination use article and the execution status of the procedure.

## Description

### BACKGROUND OF THE INVENTION

### 1. Field of the Invention

The present invention relates to an endoscopic examination information analysis apparatus and an endoscopic examination information analysis method for analyzing information related to an examination using an endoscope, and an endoscopic examination information input support system using the endoscopic examination information analysis apparatus.

### 2. Description of the Related Art

In a case where an examination using an endoscope is performed in a medical institution such as a hospital, the examination is managed based on information called an "examination order", and processing in the hospital including the actual examination is generally performed on the information of this examination order. In the examination order, information on a procedure, medicine, material, and the like to be used for the examination may be input as individual examination information generated for each examination of a patient. It is necessary to input information of "which of procedure, medicine, material, and the like are actually used for the examination" in reporting the execution of the examination, and payment processing is performed based on this information.

Since it takes time and effort to input information in execution reports and the like over a wide variety of articles and the like to be used in the examination, it is known to support the input of information related to the examination. For example, JP2011-076465A describes that the input of the information is supported by extracting and displaying candidates for the examination use article while referring to a database in which medicines, medical materials, and the like are registered.

### SUMMARY OF THE INVENTION

In a case where the input of the information is supported based on the candidates for the examination use article and the like, the "examination use article and the like are registered in the database and are extracted and displayed as the candidates but are not used in the actual examination", or the "examination use articles and the like are not registered and are not extracted and displayed as the candidates but are used in the actual examination". In these cases, it is necessary to input medicines or the like that are not extracted and displayed as the candidates whenever the examination is performed, or it is necessary to exclude the input of the medicines or the like that are extracted and displayed as the candidates. As described above, when the registered content of the database and the candidates for the examination use article or the like that are extracted and displayed are not appropriate, a work load of the input of the information increases. However, JP2011-076465A does not consider these problems, and is not sufficient to support the input of the examination information.

The inventors of the present application have made extensive studies on the above-mentioned problems, and have found that "appropriate candidates for the examination use article or the like can be presented in the case of inputting the individual examination information by analyzing the examination information and grasping a use status of the examination use article, and the input of endoscopic examination information can be efficiently supported".

The present invention has been made in view of such circumstances, and an object of the present invention is to provide an endoscopic examination information analysis apparatus and an endoscopic examination information analysis method capable of enabling a user to easily grasp a use status of an examination use article and an execution status of a procedure, and an endoscopic examination information input support system capable of efficiently supporting an input of examination information.

In order to achieve the aforementioned object, an endoscopic examination information analysis apparatus according to a first aspect of the present invention comprises an examination database in which at least ones of examination use articles used in an examination using an endoscope or procedures executed in the examination are recorded in association with a plurality of examination items of the examination, an examination information acquisition unit that acquires examination information which is generated whenever the examination is executed and indicates the examination use article in the examination and/or the procedure in the examination, a database update unit that updates the examination database based on the examination information, an examination item selection unit that selects an examination item from among the plurality of examination items recorded in the examination database, an information calculation unit that calculates use information of the examination use article based on a use history of the examination use article, and calculates execution information of the procedure based on an execution history of the procedure for the selected examination item, and a display controller that displays the calculated use information and execution information on a display device for each examination use article or procedure.

In the first aspect, the examination database is updated based on the examination information generated whenever the examination is executed. Since the use information of the examination use article is calculated based on the use history for the selected examination item, the execution information of the procedure is calculated based on the execution history, and the calculated use information and execution information are displayed on a display device for each examination use article or procedure, the user can easily grasp the use status of the examination use article and the execution status of the procedure.

In the first aspect and the following aspects, the "use history" and the "execution history" may include identification information and execution date and time of the examination used for the examination use article and the procedure. The "use information" can be expressed in various formats such as the number of times of use, a use rate, and the amount of use of the examination use article, and the "execution information" can be expressed in various formats such as the number of times of execution and an execution rate of the procedure. The use information and the execution information may be displayed according to a use degree or an execution degree (for example, in descending order or ascending order of the use degree or the execution degree). Devices for realizing the first aspect may be housed in one housing or may be housed in a plurality of housings. These devices may be installed in a single facility such as one room, or devices installed in a plurality of facilities may be connected via a network.

In the first aspect, in an endoscopic examination information analysis apparatus according to a second aspect, the examination database records the use history for a designated use article which is the examination use article designated to be used in advance and the execution history for a designated execution procedure which is the procedure designated to be executed in advance, and the use history for a selected use article which is the examination use article to be used by selection and the execution history for a selected execution procedure which is the procedure designated to be executed by selection, the database update unit updates the use history for the designated use article and the execution history for the designated execution procedure, and the use history for the selected use article and the execution history for the selected execution procedure, and the display controller displays the use information for the designated use article and the execution information for the designated execution procedure for the selected examination item, and the use information for the selected use article and the execution information for the selected execution procedure for the selected examination item with discrimination from each other.

In the second aspect, for example, the examination use article and the procedure which are highly likely to be used in individual examinations or are initially set (as defaults) to be used can be registered as the designated use article and the designated execution procedure. In the second aspect, since the use information for the designated use article and the execution information for the designated execution procedure, and the use information for the selected use article and the execution information for the selected execution procedure are displayed with discrimination from each other, the user can efficiently analyze, for example, "whether or not the designated use article and the designated execution procedure are appropriately registered" by comparing the use information of the registered examination use article and the execution information of the registered procedure with the use information of another examination use article and the execution information of another procedure.

In the second aspect, an endoscopic examination information analysis apparatus according to a third aspect further comprises a list editing unit that edits a list of the designated use article and the designated execution procedure. In the third aspect, it is possible to edit an appropriate list by reflecting analysis results of the designated use article and the designated execution procedure described above on the second aspect. In the third aspect, it is possible to edit the list according to the instruction of the user.

In the third aspect, in an endoscopic examination information analysis apparatus according to a fourth aspect, the display controller displays information prompting the list to be edited based on the use information and/or the execution information on the display device. According to the fourth aspect, the user can efficiently perform the analysis by editing the list based on the displayed information. In the fourth aspect, for example, in a case where there is the examination use article having the low use degree among the examination use articles registered in the list, or in a case where there is the examination use article that is not registered in the list but has the high actual use degree, information prompting the list to be edited can be displayed (the same applies to the execution degree of the procedure). The display of the information prompting the list to be edited can be performed according to, for example, "whether or not the use degree (or execution degree) is higher or lower than a threshold value".

In any one of the first to fourth aspects, an endoscopic examination information analysis apparatus according to a fifth aspect, the display controller displays the use information for the examination use article included in an execution set which is information indicating a combination of the examination use article and the procedure and the execution information for the procedure included in the execution set with discrimination from the use information for the examination use article which is not included in the execution set and the execution information for the procedure which is not included in the execution set. According to the fifth aspect, for example, a situation of "there is the examination use article having the low use degree among the examination use articles registered in the execution set" or "there is the examination use article that is not registered in the execution set but has the high actual use degree" can be grasped (the same applies to the execution degree of the procedure). The execution set may be defined so as to correspond to each of the plurality of examination items, or may not correspond to the examination items.

In the fifth aspect, an endoscopic examination information analysis apparatus according to a sixth aspect further comprises an execution set editing unit that edits the combination of the examination use article and the procedure in the execution set. In the sixth aspect, for example, the processing of "excluding the examination use article that is registered in the execution set and has the low use degree from the execution set" or "adding the examination use article that is not registered in the execution set but has the high use degree to the execution set" can be performed according to the instruction of the user (the same applies to the execution degree of the procedure).

In the sixth aspect, an endoscopic examination information analysis apparatus according to a seventh aspect, the display controller displays information prompting the combination to be edited based on the use information and the execution information on the display device. In the seventh aspect, for example, in a case where there is the examination use article having the low use degree among the examination use article registered in the combination (execution set) or in a case where there is the examination use article that is not registered in the combination but has the high use degree, the information prompting the combination (execution set) to be edited can be displayed (the same applies to the execution degree of the procedure).

In any one of the first to seventh aspects, in an endoscopic examination information analysis apparatus according to an eighth aspect, the examination information acquisition unit acquires the examination information from a plurality of medical institutions, and the information calculation unit calculates the use information and the execution information independently for each of the plurality of medical institutions. The eighth aspect can be adopted in a case where there is an attempt to independently calculate and manage the use information and the execution information for each medical institution such as a hospital.

In any one of the first to seventh aspects, in an endoscopic examination information analysis apparatus according to a ninth aspect, the examination information acquisition unit acquires the examination information from a plurality of medical institutions, and the information calculation unit calculates the use information and the execution information over two or more medical institutions of the plurality of medical institutions. In the ninth aspect, for example, the use information and the execution information can be calculated and shared among a plurality of medical institutions such as group-related hospitals.

In any one of the first to ninth aspects, an endoscopic examination information analysis apparatus according to a tenth aspect further comprises an update condition setting unit that sets an update condition of the examination database. The database update unit updates the examination database based on the set update condition. In the tenth aspect, conditions in which the "use history and/or the execution history are updated whenever the examination information is acquired", the "use history and/or the execution history are updated every hour", the "use history and/or the execution history are updated every day", and the "use history and/or the execution history are updated every week" can be set. The specific condition may be set according to the designation of the user.

In any one of the first to tenth aspects, in an endoscopic examination information analysis apparatus according to an eleventh aspect, the information calculation unit calculates, as the use information, a use rate expressed by a ratio of a total number of times of use for each medicine and medical material included in the examination use article to a total number of times of execution of the examination, and calculates, as the execution information, an execution rate expressed by a ratio of a total number of times of execution of the procedure to a total number of times of execution of the examination. The eleventh aspect defines specific aspects of the use information and the execution information.

In any one of the first to eleventh aspects, an endoscopic examination information analysis apparatus according to a twelfth aspect, the examination use article includes at least one of a medicine or a medical material. The twelfth aspect defines specific aspects of the examination use article.

In any one of the first to twelfth aspects, in an endoscopic examination information analysis apparatus according to a thirteenth aspect, the display controller displays the calculated use information for each medicine and medical material on the display device. According to the thirteenth aspect, the user can easily grasp the use information for each medicine and medical material.

In order to achieve the aforementioned object, an endoscopic examination information input support system according to a fourteenth aspect of the present invention comprises the endoscopic examination information analysis apparatus according to any one of the first to thirteenth aspects, the display device, a candidate extraction unit that extracts candidates for the examination use article to be used in the examination while referring to the examination database, and displays the extracted candidates for the examination use article on the display device, and an instruction input unit that inputs an instruction to register, as the examination information, at least a part of the displayed candidates. The candidate extraction unit extracts the candidates for the examination use article to be used in the examination while referring to the examination database based on at least one of the examination item selected from among the plurality of examination items or the selected procedure.

In accordance with the endoscopic examination information input system according to the fourteenth aspect, the user can grasp the use status of the examination use article and the execution status of the procedure by the endoscopic examination information analysis apparatus according to any one of the first to thirteenth aspects. Since the candidates for the examination use article are extracted and displayed while referring to the examination database updated based on the examination information and the instruction to register at least a part of the displayed candidates as the examination information is input, it is possible to efficiently support the input of the examination information based on the appropriate candidates.

In order to achieve the aforementioned object, an endoscopic examination information analysis method according to a fifteenth aspect of the present invention comprises a recording step of recording at least ones of examination use articles used in an examination using an endoscope or procedures executed in the examination in association with a plurality of examination items of the examination in an examination database, an examination information acquisition step of acquiring examination information which is generated whenever the examination is executed and indicates the examination use article in the examination and/or the procedure in the examination, a database update step of updating the examination database based on the acquired examination information, an examination item selection step of selecting an examination item from among the plurality of examination items recorded in the examination database, an information calculation step of calculating use information of the examination use article based on a use history of the examination use article and calculating execution information of the procedure based on an execution history of the procedure for the selected examination item, and a display control step of displaying the calculated use information and execution information on a display device for each examination use article or procedure. According to the thirteenth aspect, the user can easily grasp the use status of the examination use article and the execution status of the procedure similarly to the first aspect.

In the fifteenth aspect, an endoscopic examination information analysis method according to a sixteenth aspect, in the recording step, at least ones of medicines or medical materials are recorded as the examination use articles.

In the fifteenth or sixteenth aspect, an endoscopic examination information analysis method according to a seventeenth aspect, in the display control step, the calculated use information is displayed on the display device for each medicine and medical material.

The endoscopic examination information analysis method according to the fifteenth to seventeenth aspects may further include the same configuration as the second to eleventh aspects. A program causing an endoscopic examination information analysis apparatus to execute the endoscopic examination information analysis method according to these aspects, and a non-transitory recording medium recording a computer-readable code of the program can also be included as the aspects of the present invention.

As described above, in accordance with the endoscopic examination information analysis apparatus and the endoscopic examination information analysis method according to the embodiment of the present invention, the user can easily grasp the use status of the examination use article and the execution status of the procedure. In accordance with the endoscopic examination information input support system according to the embodiment of the present invention, it is possible to efficiently support the input of the examination information.

### BRIEF DESCRIPTION OF THE DRAWINGS

Fig. 1 is a diagram showing a schematic configuration of an endoscopic examination system.
Fig. 2 is a diagram showing a functional configuration of a client computer.
Fig. 3 is a diagram showing a functional configuration of a cloud server.
Fig. 4 is a diagram showing a scene in which information is recorded and a history is updated in an examination database.
Fig. 5 is a diagram showing a scene in which display conditions are set.
Fig. 6 is another diagram showing the scene in which the display conditions are set.
Figs. 7A and 7B are diagrams showing a scene in which a use rate and an execution rate are calculated.
Fig. 8 is a diagram illustrating an example of a graph of a simultaneous execution rate.
Fig. 9 is a diagram showing a scene in which details of a procedure are selected.
Fig. 10 is a diagram showing another example of the graph of the simultaneous execution rate.
Fig. 11 is a diagram showing a scene in which an execution set is selected.
Fig. 12 is a diagram showing a scene in which a message prompting a list to be edited is displayed.
Fig. 13 is a diagram showing an example of an execution rate of an item registered in the execution set.

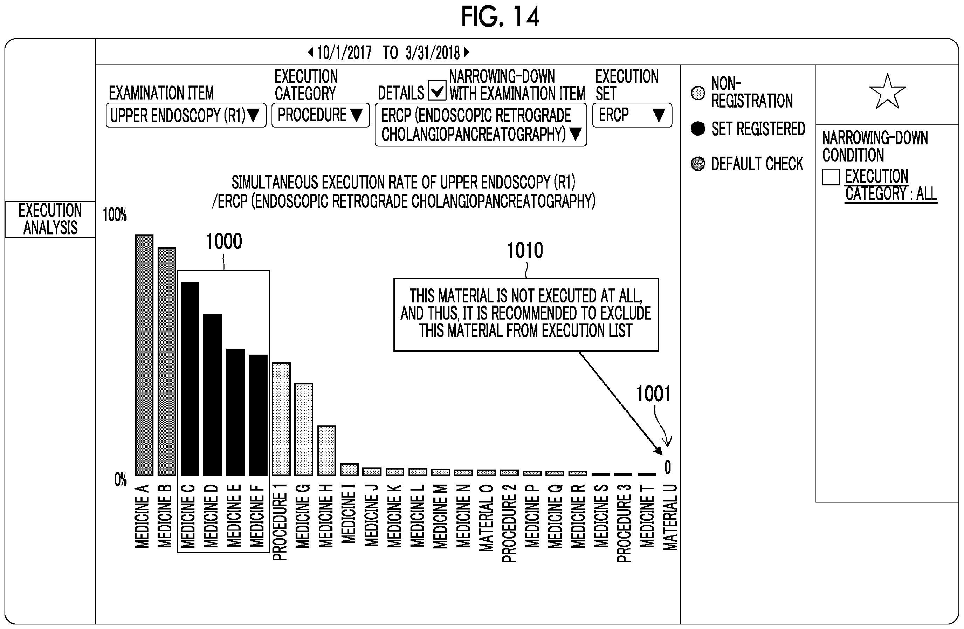

Fig. 14 is a diagram showing a scene in which a message prompting the execution set to be edited is displayed.
Fig. 15 is a diagram showing a scene in which the procedure is associated with the examination item.
Fig. 16 is another diagram showing a scene in which the procedure is associated with the examination item.
Fig. 17 is a diagram showing a scene in which a medicine is associated with the examination item.
Fig. 18 is another diagram showing a scene in which the medicine is associated with the examination item.
Fig. 19 is a diagram showing a scene in which the procedure is associated with the medicine.
Fig. 20 is a diagram showing a scene in which the execution set is newly registered.
Fig. 21 is another diagram showing a scene in which the execution set is newly registered.
Fig. 22 is still another diagram showing a scene in which the execution set is newly registered.
Fig. 23 is a diagram showing a scene in which the procedure, medicine, and material included in the execution set are selected.
Fig. 24 is another diagram showing a scene in which the procedure, medicine, and material included in the execution set are selected.
Fig. 25 is still another diagram showing a scene in which the execution set is newly registered.
Fig. 26 is a diagram showing a scene in which the newly registered execution set is displayed.
Fig. 27 is a diagram showing a scene in which the execution set is edited.
Fig. 28 is another diagram showing a scene in which the execution set is edited.
Fig. 29 is a diagram showing a scene in which the execution is reported.
Fig. 30 is another diagram showing a scene in which the execution is reported.
Fig. 31 is a diagram showing a scene in which the execution set is selected.
Fig. 32 is a diagram showing a scene in which candidates for the procedure, medicine, and material are extracted and displayed.

### DESCRIPTION OF THE PREFERRED EMBODIMENTS

Hereinafter, an embodiment of an endoscopic examination information analysis apparatus, an endoscopic examination information input support system, and an endoscopic examination information analysis method according to the embodiment of the present invention will be described in detail with reference to the accompanying drawings.

### <First Embodiment>

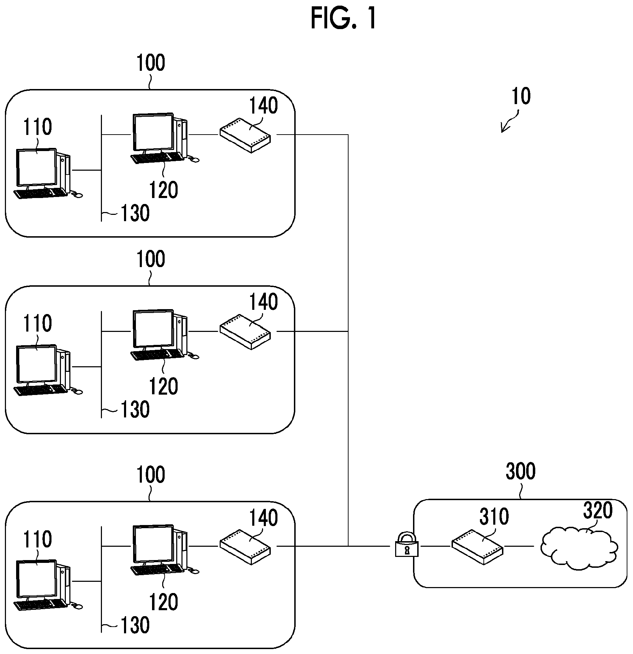

Fig. 1 is a diagram showing a schematic configuration of an endoscopic examination system 10 (endoscopic examination information analysis apparatus an endoscopic examination information input support system). The endoscopic examination system 10 includes in-hospital systems 100 belonging to a hospital layer and a cloud server section 300 (endoscopic examination information analysis apparatus and endoscopic examination information input support system).

### <Configuration of in-hospital system>

The in-hospital system 100 includes a client computer 110 and an in-hospital server 120. The in-hospital system 100 may include a hospital information system (HIS), a picture archiving and communication system (PACS), a radiology information system (RIS), and the like which are not shown. These systems are connected via an in-hospital LAN 130 (LAN: Local Area Network) and a router 140 to mutually transmit and receive necessary information. The endoscopic examination system 10 may include one or more in-hospital systems 100. In the in-hospital system 100, items such as "execution analysis" are analyzed by the cloud server section 300, and the results are displayed by the client computer 110. Specifically, in the in-hospital system 100, "patient waiting time", "examination required time", "examination number", "execution input time", "execution analysis", "report time", "washing number", "scope operation time", and the like can be aggregated on various analysis axes, can be drilled down (items to be aggregated are further refined by drilling down an aggregation level of data), and can be sorted. Examples of the analysis axis include an age, a gender, an imaging location such as an imaging room, an imaging engineer, imaging date, and an examination time zone.

### <Configuration of client computer>

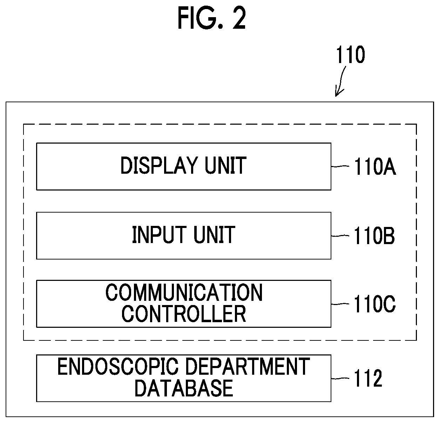

The client computer 110 is included in an endoscopic department system (not shown), and includes a display unit 110A (display device), an input unit 110B, a communication controller 110C, and an endoscopic department database 112 as shown in Fig. 2. The display unit 110A is a device such as a liquid crystal display, and displays endoscopic examination information, an analysis result thereof, information on an examination database to be described later, and the like. The input unit 110B is an input device such as a mouse and a keyboard (not shown), and a user can input information necessary for analyzing the endoscopic examination information, registering examination information, and the like via the input unit 110B. The communication controller 110C controls communication with the other systems of the in-hospital system 100 and communication with the cloud server section 300 via the in-hospital server 120. Functions of the client computer 110 can be realized by using various processors and storage devices as will be described later regarding the cloud server 320.

### <Configuration of cloud server section>

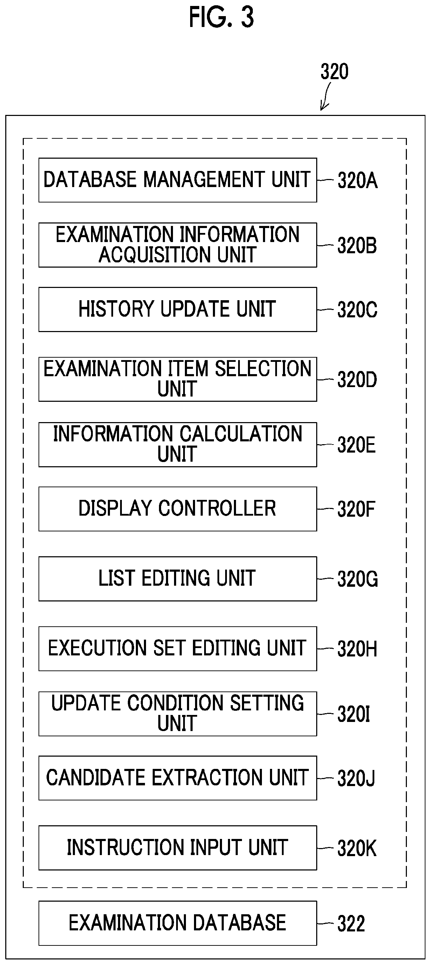

The cloud server section 300 (endoscopic examination information analysis apparatus and endoscopic examination information input support system) includes a router 310 and a cloud server 320, and communicates with the in-hospital systems 100. The cloud server 320 receives information necessary for the analysis and/or input support of the endoscopic examination information from the client computers 110 of the in-hospital systems 100, performs processing such as calculation of use information and/or execution information and extraction of input candidates, and the like, transmits the results to the client computer 110, and displays the results on the display unit 110A. Fig. 3 is a diagram showing a functional configuration of the cloud server 320. The cloud server 320 includes a database management unit 320A, an examination information acquisition unit 320B, a database update unit 320C, an examination item selection unit 320D, an information calculation unit 320E, a display controller 320F, a list editing unit 320G, an execution set editing unit 320H, an update condition setting unit 3201, a candidate extraction unit 320J, and an instruction input unit 320K. The cloud server 320 also includes an examination database 322.

Functions of the cloud server 320 described above may be realized by using various processors. The various processors include, for example, a central processing unit (CPU) which is a general-purpose processor that realizes various functions by executing software (program). The various processors described above include a graphics processing unit (GPU) which is a processor specialized in imaging processing and a programmable logic device (PLD) which is a processor capable of changing a circuit configuration after a field programmable gate array (FPGA). A dedicated electric circuitry which is a processor having a circuit configuration specifically designed to execute specific processing such as an application specific integrated circuit (ASIC) and the like are also included in the various processors described above.

The function of each unit may be realized by one processor, or may be realized by a plurality of processors of the same type or different types (for example, a plurality of FPGAs, a combination of a CPU and an FPGA, or a combination of a CPU and a GPU). A plurality of functions may be realized by one processor. As an example in which the plurality of functions is realized by one processor, firstly, there is a form in which one processor is constituted by a combination of one or more CPUs and software and this processor is realized as the plurality of functions as represented by a computer. Secondly, there is a form in which a processor that realizes the functions of the entire system by one integrated circuit (IC) chip is used as represented by a system on chip (SoC). As described above, various functions are realized by using one or more of the various processors described above as a hardware structure. The hardware structure of these various processors is, more specifically, an electric circuitry in which circuit elements such as semiconductor elements are combined.

In a case where the processor or electric circuitry described above executes software (program), a processor (computer) readable code of the software to be executed is stored in a non-transitory recording medium such as a read only memory (ROM), and the processor refers to this software. The software stored in the non-transitory recording medium includes a program for executing an endoscopic examination information analysis method and an endoscopic examination information input support method according to the embodiment of the present invention. The code may be recorded in a non-transitory recording medium such as various magneto-optical recording devices and semiconductor memories instead of the ROM. For example, a random access memory (RAM) is used as a transitory storage region in the case of processing using the software, and data stored in an electronically erasable and programmable read only memory (EEPROM) (not shown) may be referred to.

The examination database 322 includes various kinds of magneto-optical recording devices and/or semiconductor recording devices.

### <Analysis of endoscopic examination information>

Processing of endoscopic examination information analysis in the endoscopic examination system 10 having the above-described configuration will be described.

### <Configuration of examination database and acquisition of examination information>

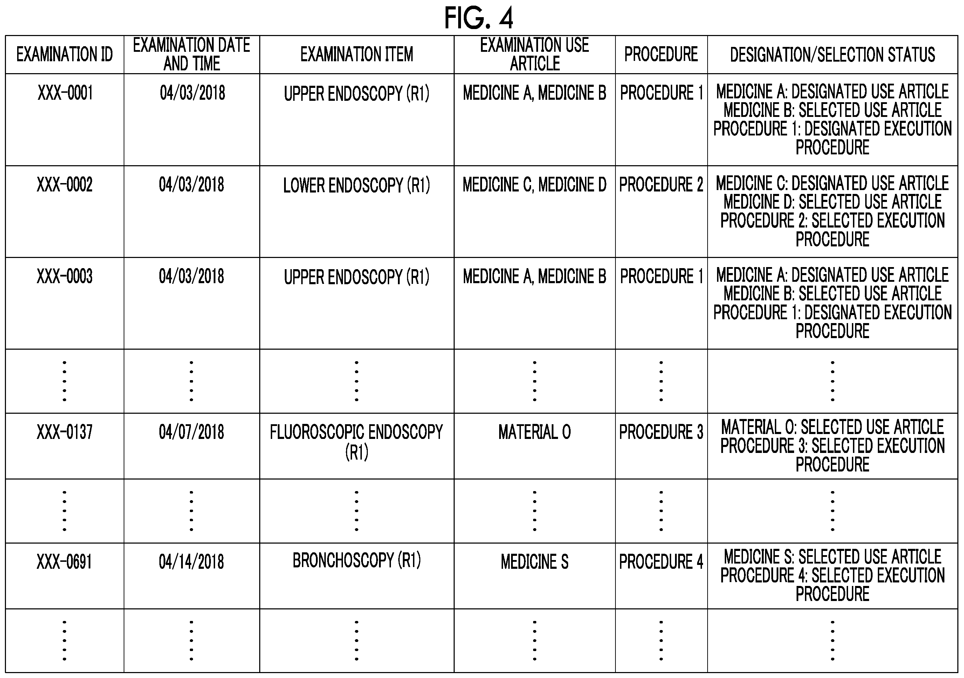

Fig. 4 is a table showing an example of a data structure of the examination database 322 (examination database). In the examination database 322, at least ones of examination use articles (including medicines or materials (medical materials)) used in an examination or the procedures executed in the examination are recorded in association with a plurality of examination items of the examination using an endoscope (recording step). Such information is recorded and/or read out by the management of the database management unit 320A. In the examination database 322, examinations having the same field of "examination item" but different examination IDs are handled as "examination item is one". For example, in the example of Fig. 4, an examination item "upper endoscopy (R1)" corresponds two examination IDs (examination IDs are "XXX-0001" and "XXX-0003") and an examination item "lower endoscopy (R1)" corresponds to one examination ID (examination ID is "XXX-0002") in a range of the examination IDs "XXX-0001" to "XXX-0003", but it is assumed that "there are two examination items of 'upper endoscopy (Rl)' and 'lower endoscopy (R1)'" in this range. The examination database 322 records a use history of a designated use article which is the examination use article designated to be used in advance and an execution history of a designated execution procedure which is a procedure designated to be executed in advance, and a use history of a selected use article which is the examination use article to be used by selection and an execution history of a selected execution procedure which is a procedure designated to be executed by the selection. For example, as shown in the rightmost column of Fig. 4, whether the examination use article and procedure used in each examination are the designated use article and the designated execution procedure or the selected use article and the selected execution procedure in association with the examination item. The examination database 322 may independently record data as shown in Fig. 4 for each examination item (for example, for one examination item such as "upper endoscopy (R1)"). The examination database 322 may record a table that defines whether the examination use articles and procedures are the designated use articles (designated execution procedures) or the selected use articles (selected execution procedures) separately from the data shown in Fig. 4. A list of the designated use articles which are the examination use articles designated to be used in advance and the designated execution procedures which are the procedures designated to be executed in advance (default check items) and an execution set which is information indicating a combination of the examination use article and procedure are also recorded in the examination database 322.

The examination information acquisition unit 320B (examination information acquisition unit) acquires examination information indicating the examination use article in the examination and/or the procedure in the examination generated whenever the examination is executed by the in-hospital system 100 (examination information acquisition step), and the database management unit 320A records the examination information in the examination database 322 (recording step). The examination information includes, for example, an examination ID (ID: Identification), an examination item, an examination date and time, information on the examination use article and/or procedure, corresponds to the data in each row in the table of Fig. 4, and may include, information, other information such as a reception date and time and a patient ID.

### <Update of examination database>

The database update unit 320C (database update unit) updates the examination database 322 based on the examination information acquired by the examination information acquisition unit 320B (database update step). Specifically, the database update unit 320C updates the list illustrated in Fig. 4 based on the acquired examination information. As the update condition of the examination database 322, the update condition setting unit 3201 can set any period or timing such as "every time examination is performed", "every hour", "every day", "every week". These update conditions may be set depending on an input of the user via the client computer 110.

### <Setting display conditions of use rate and execution rate>

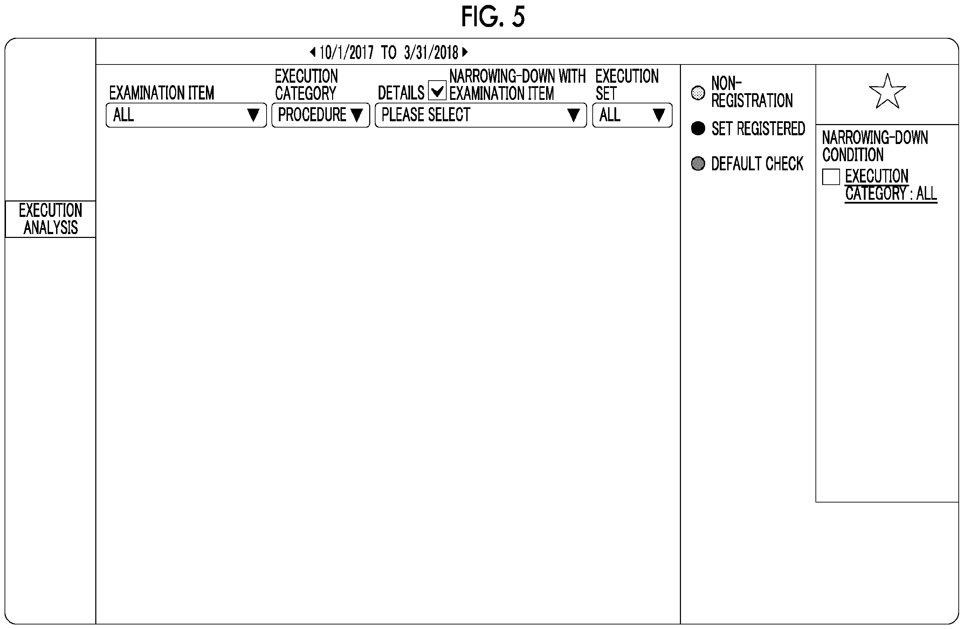

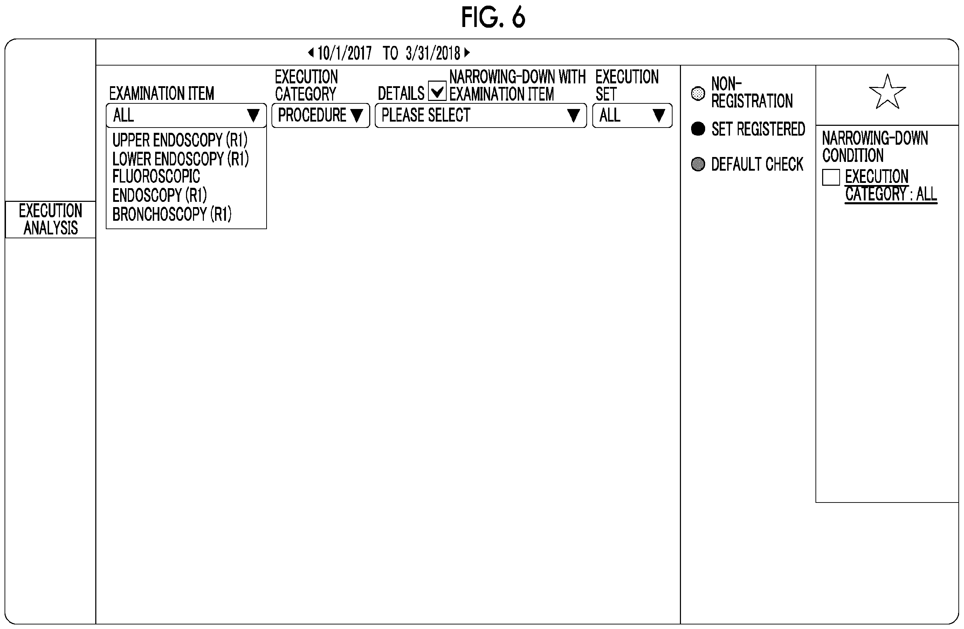

In the endoscopic examination system 10, a use rate and an execution rate are displayed based on the use history and the execution history described above. Figs. 5 and 6 are diagrams showing examples of screens (screens displayed on the display unit 110A by the information calculation unit 320E, the display controller 320F, and the like) for describing a scene in which display conditions are set, and the display condition is set by selecting a pull-down item on the screen examples. Specifically, in a case where the item is changed in the pull-down menu of "examination item", a simultaneous execution rate (use rate and execution rate; to be described later) of the examinations corresponding to newly selected "examination item" and "details" is displayed in a graph. In a case where the item is selected in the pull-down menu of "execution category", the item displayed in the pull-down menu of "details" is switched according to this selection. The currently selected "details" is in a non-selection state. In a case where the item is changed in the pull-down menu of "details", the simultaneous execution rate of the examinations corresponding to the newly selected "details" and "examination item" is displayed. In a case where "narrowing-down with examination item" is checked, the item displayed in the pull-down of "details" is only the item associated with the examination item. In a case where the item is changed in the pull-down menu of "execution set", the items (procedures, medicines, and materials) included in the selected execution set are displayed as "set registered" with discrimination from each other in the graph of the simultaneous execution rate, and the items that have not been executed are also displayed.

### <Selection of examination item>

Under the configuration described above, the user selects the examination item from among the plurality of examination items by the input unit 110B via the screen displayed on the display unit 110A of the client computer 110. Similarly, the user selects a processing target period. Fig. 5 is a diagram showing a scene in which a target period (here, from October 1, 2017 to March 31, 2018) is selected at the uppermost portion of the screen, and Fig. 6 shows a diagram showing a scene in which the user selects the examination item from among the plurality of examination items (upper endoscopy (R1), lower endoscopy (R1), fluoroscopic endoscopy (R1), bronchoscopy (R1)) in the pull-down menu of "examination item" present at an upper portion of the screen. The examination item selection unit 320D (examination item selection unit) selects the examination item from among the plurality of examination items according to the operation of the user described above (examination item selection step). In the following example, it is assumed that "upper endoscopy (R1)" is selected as the examination item. The examination item selection unit 320D similarly selects the processing target period according to the operation of the user. In Figs. 5 and 6, the graphs of the use rate and the execution rate, and the like which will be described later are not displayed until the examination item or the like is selected.

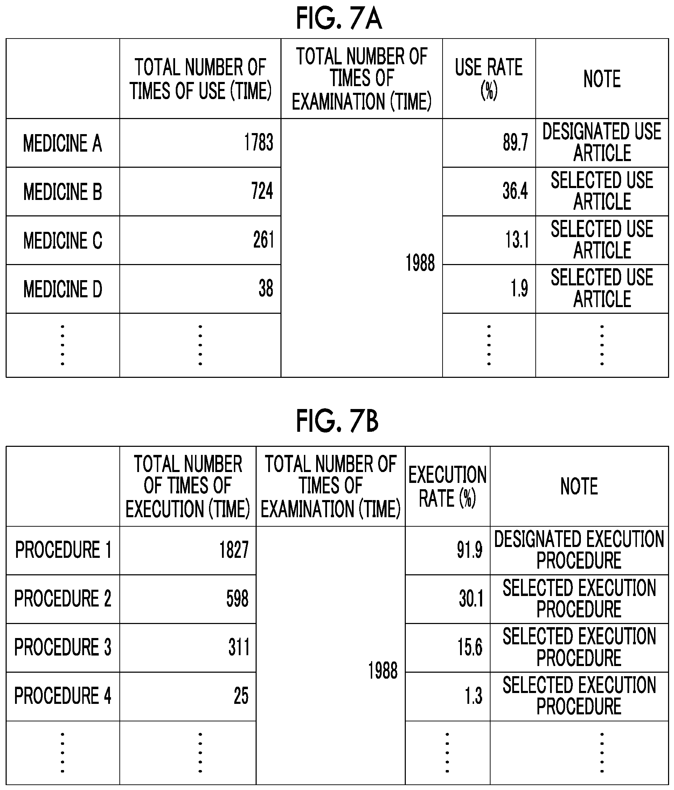

The information calculation unit 320E (information calculation unit) calculates use information of the examination use article based on the use history of the examination use article for the selected examination item "upper endoscopy (R1)", and calculates execution information based on the execution history of the procedure (information calculation step). The information calculation unit 320E obtains the number of times of the use and the number of times of the execution by aggregating information on the use history and the execution history recorded as shown in Fig. 4 for each medicine, medical material, and procedure. The information calculation unit 320E calculates, as the use information, the use rate expressed by a ratio of the total number of times of the use for each medicine and medical material included in the examination use article to the total number of times of the execution of the examination, and calculates, as the execution information, the execution rate expressed by a ratio of the total number of times of the execution of the procedure to the total number of times of the execution of the examination. Specifically, the information calculation unit 320E adds the number of times of the use for each medicine and medical material, and calculates, as the use information, the use rate expressed by the ratio of the total number of times of the use for each medicine and medical material to the total number of times of the execution of the examination. In the examples of Figs. 7A and 7B, since the total number of times of the examination is 1988 times and the total number of times of the use of medicine A is 1783 times, the use rate is 1788/1988 = 0.897 (89.7%). The execution rate can be similarly calculated for the procedure, and the execution rate for procedure 1 is 0.919 (91.9%).

### <Display of use rate and execution rate for medicine and procedure>

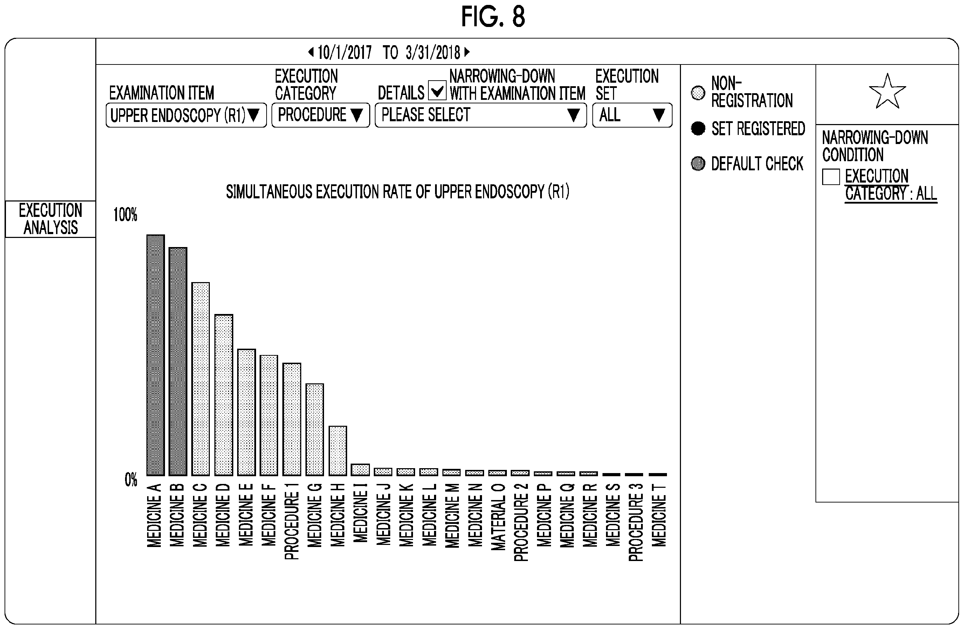

The display controller 320F (display control step) displays the use rate and the execution rate calculated by the information calculation unit 320E for the selected examination item (here, upper endoscopy (R1)) for each medicine and medical material included in the examination use article and for each procedure in descending order of the use rate or the execution rate on the display unit 110A (display device) of the client computer 110 as shown in the example of Fig. 8 (display control step). Due to the display as shown in Fig. 8, the user can easily grasp a use status of the examination use article and an execution status of the procedure. The display controller 320F may display the use rate or the execution rate in ascending order contrary to the mode of Fig. 8. The user can also easily grasp the use status of the examination use article and the execution status of the procedure due to such a display mode, and it is effective for extracting the medicine or the like with a low use rate or a low execution rate (or the designation of the use or execution is excluded). Whether to display the use rate or the execution rate in ascending order or in descending order may be set according to the instruction of the user via the input unit 110B of the client computer 110.

In the graph of Fig. 8, two items (medicine A and medicine B) at a left edge of a lateral axis are articles registered as the "designated use articles" for the examination item (upper endoscopy (R1)) in the examination database 322 and the display controller 320F displays the use rates of these designated use articles with discrimination from the use rates of other use articles (selected use articles) (in this case, using different colors). The user can efficiently analyze "whether the designated use article and designated execution procedure (default check items) are appropriately registered" by comparing the use information of the designated use article and the execution information of the designated execution procedure with the use information of the selected examination use article and the execution information of the selected execution procedure. In a case where the registration is inappropriate, for example, in a case where the article and/or procedure registered as the designated use article and the designated execution procedure are rarely used or executed or in a case where the unregistered article and/or procedure are often used or executed, it takes time and effort to add and delete the item in the case of reporting the examination or registering the individual examination information. Thus, in the endoscopic examination system 10 according to the first embodiment, the list editing unit 320G (list editing unit) can edit the list of the designated use articles and the designated execution procedures (default check items) according to an instruction input via the input unit 110B of the client computer 110. A specific list editing operation will be described later. In Fig. 8 and subsequent screen examples, the use rate of the examination use article and the execution rate of the procedure are collectively referred to as the "simultaneous execution rate". Hereinafter, similar to the screen example, the use rate and the execution rate of the procedure may be collectively referred to as the "simultaneous execution rate".

### <Example of narrowing-down of display conditions>

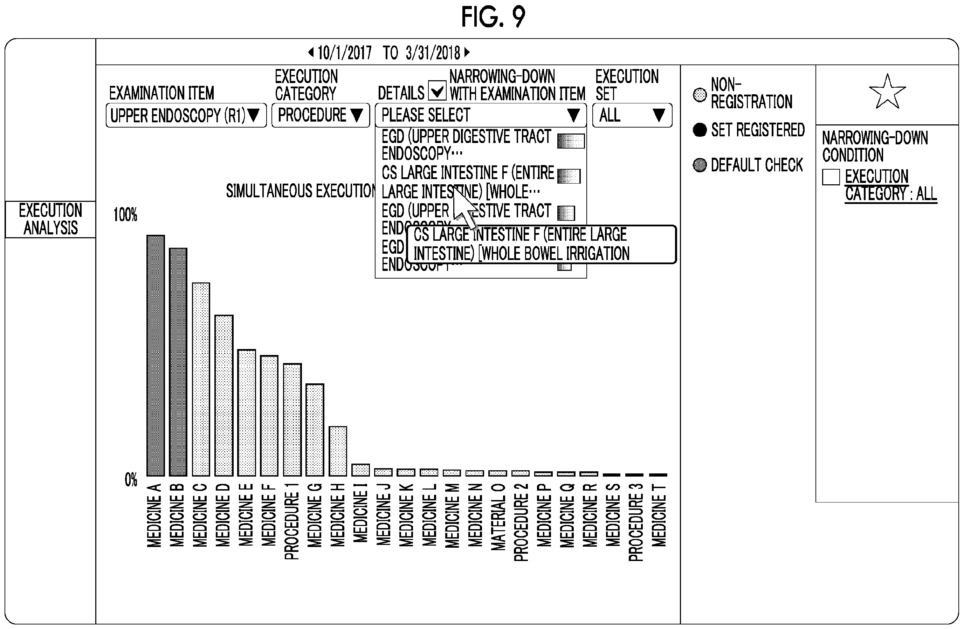

In a case where a check box of "narrowing-down with examination item" is checked at an upper portion of Fig. 8 and "procedure" is selected in the pull-down menu of "execution category", the procedure associated with the examination item is displayed in the pull-down menu of "details" regardless of whether the selected procedure is the designated execution procedure (default check item) as shown in Fig. 9. Meanwhile, in a case where the check box of "narrowing-down with examination item" is not checked, all the procedures are displayed in the field of "detail". In these cases, in a case where one of the procedures is selected in the pull-down menu of "detail", the population of the simultaneous execution rates to be displayed is changed according to the selection result. Specifically, the simultaneous execution rate of the examinations corresponding to the procedure selected in "details" and the item selected according to "examination item" is displayed. In the pull-down of "execution category", "medicine" or "material" can be selected in addition to "procedure", and the item displayed in the pull-down menu of "detail" is switched according to the selected item.

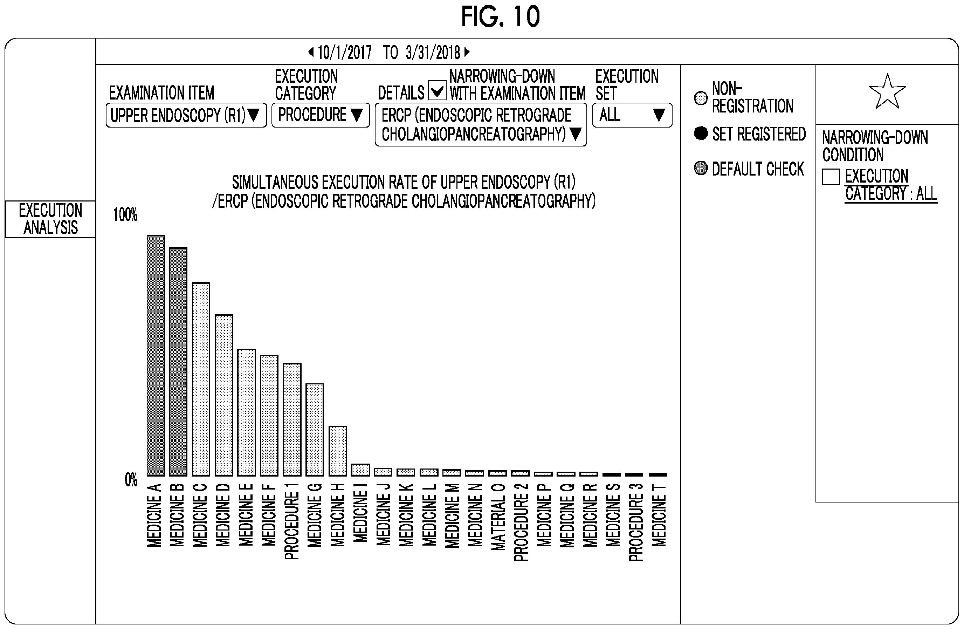

In Fig. 10, the simultaneous execution rate in a case where "upper endoscopy (R1)" is selected in the examination item, "procedure" is selected in the execution category, and "endoscopic retrograde cholangiopancreatography (ERCP)" is selected in the details (details for the procedure) is shown. In Fig. 10, two items (medicine A and medicine B) at a left edge displayed with discrimination from the other items are the designated use articles (default check items) for the examination item "upper endoscopy (R1)" or the procedure "endoscopic retrograde cholangiopancreatography (ERCP)".

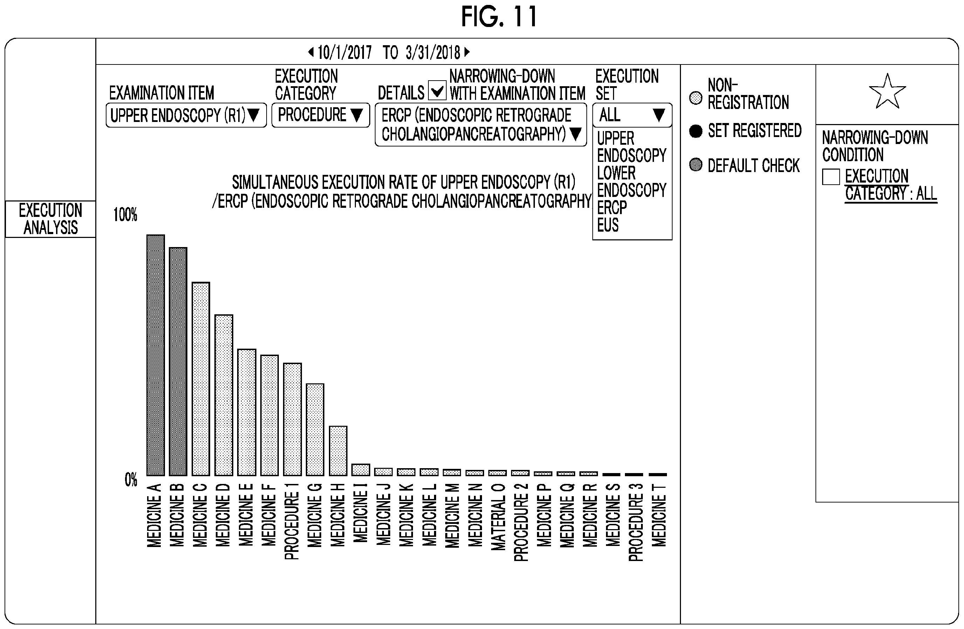

Fig. 11 is a diagram showing a scene in which the execution set is narrowed down under the same condition settings as in Fig. 10. All the execution sets (in this case, upper endoscopy, lower endoscopy, ERCP, and endoscopic ultrasonography (EUS)) are displayed in the pull-down menu of "execution set" regardless of the selection in the pull-down menu of "examination item".

### <Recommendation for editing list of designated use article and designated execution procedure>

The designation of a necessary item may be added or excluded for the registration content of the list in the case of inputting the examination information and reporting the execution by registering the designated use articles and designated execution procedures in the list (default check items). However, in a case where the content of the list is not appropriate, it takes time and effort to add the designation or exclude the designation in the case of inputting the examination information or reporting the execution. Thus, in the first embodiment, in a case where there are the article having a low use rate and/or the procedure having an execution rate among the article (medicines and medical materials) and/or the procedure (default check items) registered in the list of the designated use articles and the designated use procedures, the display controller 320F displays a message prompting the list to be edited based on the use information and/or the execution information on the display unit 110A (display device).

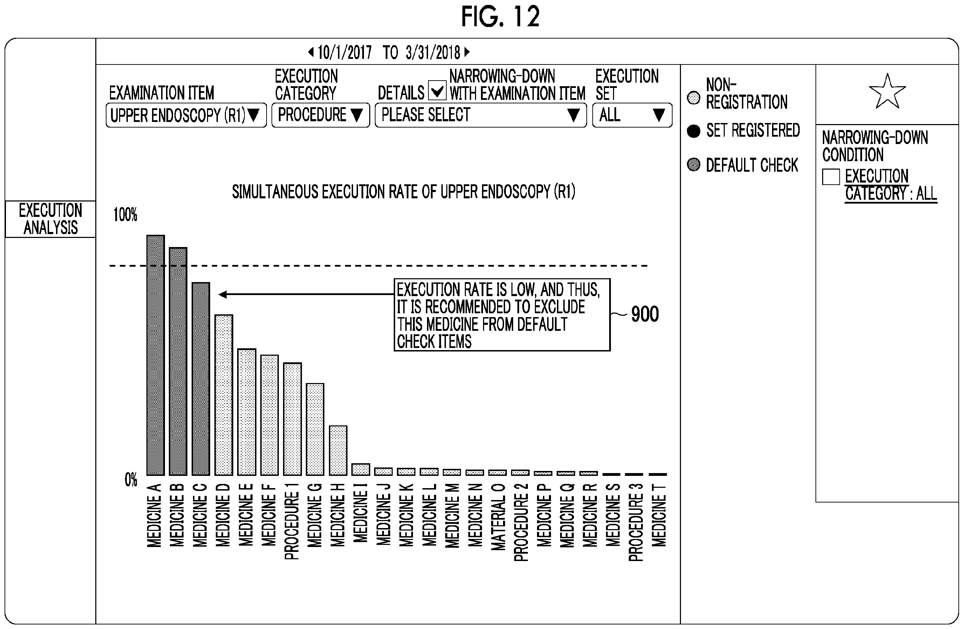

For example, in the example of Fig. 12, the third item from the left (medicine C) is registered as the designated use article in the list and the use rate is displayed with discrimination, but the use rate does not satisfy a threshold value (here, 80%, but is not limited to this value). Thus, the display controller 320F displays, for medicine C, information such as "execution rate is low, and thus, it is recommended to exclude this medicine from default check items (list of designated use articles and designated use medicines) for the upper endoscopy" (message 900) on display unit 110A. The user can give an instruction to edit the list by operating the input unit 110B, and the list editing unit 320G (list editing unit) edits the content of the list according to the instruction. A threshold value as a reference for displaying a message may be set to a value based on an instruction of the user, or may be set by the endoscopic examination system 10 regardless of the instruction of the user.

In the endoscopic examination system 10 according to the first embodiment, it is possible to easily grasp the use status of the examination use article and the execution status of the procedure by displaying the simultaneous execution rate of the examination use article and the procedure in this manner. It is possible to efficiently report the execution of the examination and register the individual examination information by editing the list of the designated use articles and designated execution procedures (default check items) while referring to the displayed execution rate.

### <Analysis and display of execution set>

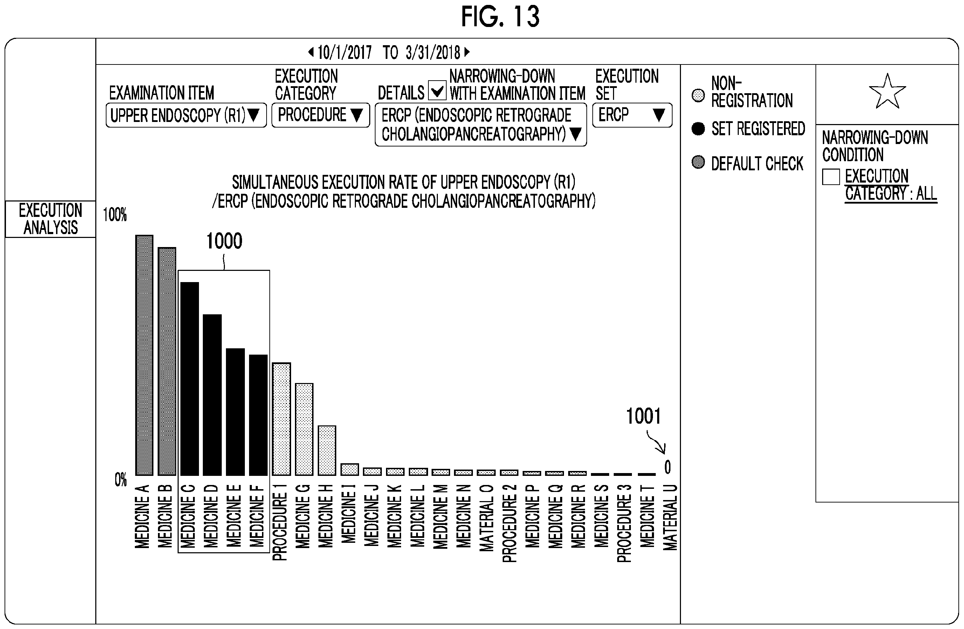

A case where the user selects the execution set "ERCP" from the pull-down menu of "execution set" at the upper portion of the screen on the screens of Figs. 8 to 11 will be described. In this case, as illustrated in Fig. 13, the display controller 320F displays the use rate (use information) of the examination use article included in the selected execution set and the execution rate (execution information) of the procedure included in the execution set with discrimination from the use rate (use information) of the examination use article that is not included in the execution set and the execution rate (execution information) of the procedure that is not included in the execution set on the display unit 110A (display device). The "execution set" is information indicating a combination of the examination use article and procedure to be used in the examination. In the example of Fig. 13, the execution set "ERCP" includes medicines C, D, E, and F surrounded by a rectangle 1000 and material U (medical material) indicated by an arrow 1001, and medicines A and B described in the list (default check items) are displayed with discrimination from the medicines and the like that are not included in the list and the execution set. A color of a label of the graph is changed for the item that is registered in the execution set but is not used or executed at all (in the example of Fig. 13, material U at a right edge and the execution rate is zero) (this item is displayed in bold letters for the sake of convenience). Accordingly, the user can easily determine "whether or not registration content of execution set is appropriate".

### <Recommendation for editing execution set>

As in the case of the list of the designated use articles and the designated execution procedures (default check items), when the content of the execution set is not appropriate, it takes time and effort to add the designation and exclude the designation in the case of inputting the examination information and reporting the execution. Thus, in the first embodiment, in a case where there are the article having the low use rate and the procedure having the execution rate among the articles (medicine and medical materials) and the procedures registered in the execution set, the display controller 320F displays information prompting the execution set to be edited based on the use information and/or the execution information on the display unit 110A (display device). For example, the display controller 320F displays, for material U that is not used at all in the example of Fig. 13, information (message 1010) such as "execution rate is low, and thus, it is recommended to exclude this material from execution list" on the display unit 110A. The user can give an editing instruction by operating the input unit 110B, and the execution set editing unit 320H (execution set editing unit) edits the content of the execution set according to this instruction. A threshold value as a reference for displaying a message may be set to a value based on an instruction of the user, or may be set by the endoscopic examination system 10 regardless of the instruction of the user.

### <Range of analysis of use information and execution information>

The collection of the examination information, the calculation and display of the information, and the like described above can be performed on the plurality of in-hospital systems 100 included in the endoscopic examination system 10. In this case, the analysis and evaluation of the information may be independently performed for each in-hospital system 100, or may be analyzed and displayed over two or more in-hospital systems 100. For example, the examination information acquisition unit 320B may acquire the examination information from the plurality of in-hospital systems 100 (medical institutions), and the information calculation unit 320E may independently calculate the use information and the execution information for each of the plurality of in-hospital systems (medical institutions). Meanwhile, the examination information acquisition unit 320B may acquire the examination information from the plurality of in-hospital systems 100 (medical institutions), and the information calculation unit 320E may calculate the use information and the execution information over two or more in-hospital systems 100 of the plurality of in-hospital systems 100 (medical institutions). In a case where the independence of the information is important, it is considered that the analysis and the like are independently performed, and in a case where there is an attempt to perform efficient analysis by sharing data collected among the plurality of medical institutions such as group-related hospitals, it is considered that the information is collected overs two or more hospitals. In a case where the information is collected and analyzed over two or more hospitals, it is possible to recommend for editing the list and the execution set based on examination information of other hospitals.

### <Association of procedure with examination item>

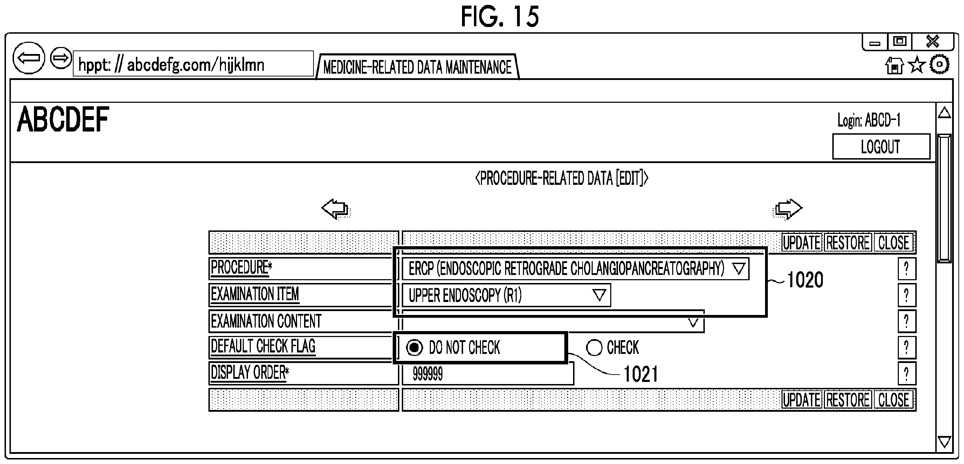

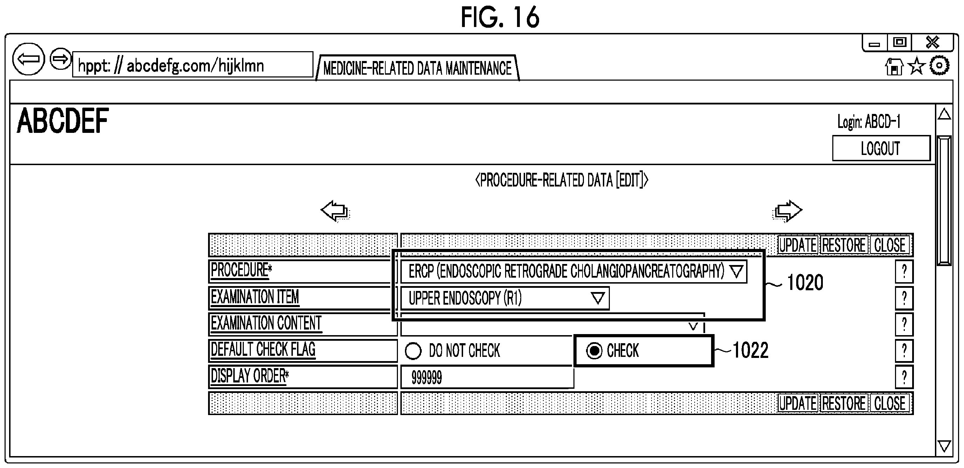

Fig. 15 is a diagram showing a scene in which the procedure is associated with the examination item. In a case where the user selects the procedure "endoscopic retrograde cholangiopancreatography (ERCP)" and the examination item "upper endoscopy (R1)" on the screen shown in Fig. 15 (within a frame 1020 of Fig. 15), the database management unit 320A associates the procedure with the examination item. In a case where "check" is selected for a default check flag, the list editing unit 320G (list editing unit) sets the procedure "ERCP" as the default check item (designated execution procedure) of the examination item "upper endoscopy (R1)", but "do not check" is selected (within a frame 1021) in the example of Fig. 15. Similarly, Fig. 16 is a diagram showing a scene in which the procedure is associated with the examination item. However, "check" is selected for the default check flag (within a frame 1022), and the list editing unit 320G sets the procedure "ERCP" as the default check item (designated execution procedure) of the examination item "upper endoscopy (R1)". Information on the association is recorded in the examination database 322. The frames in Figs. 15 and 16 are frames for description, and are not displayed on the actual screen.

### <Association of medicine with examination item>

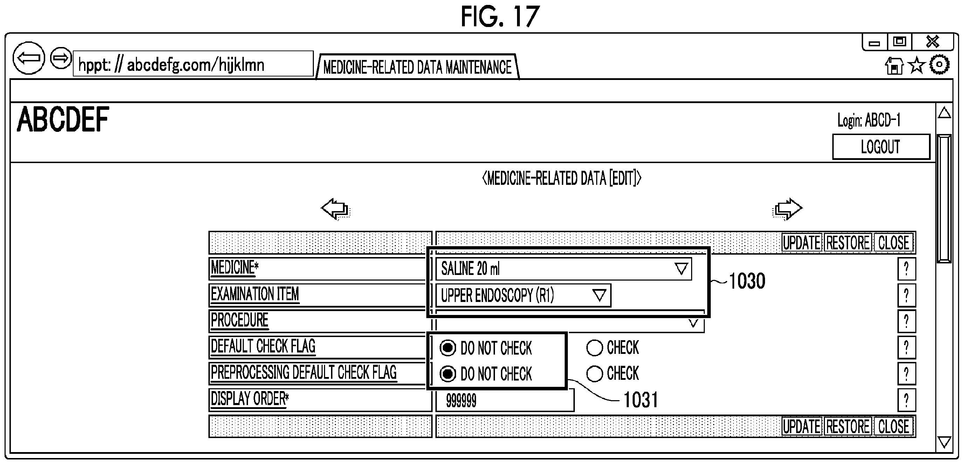

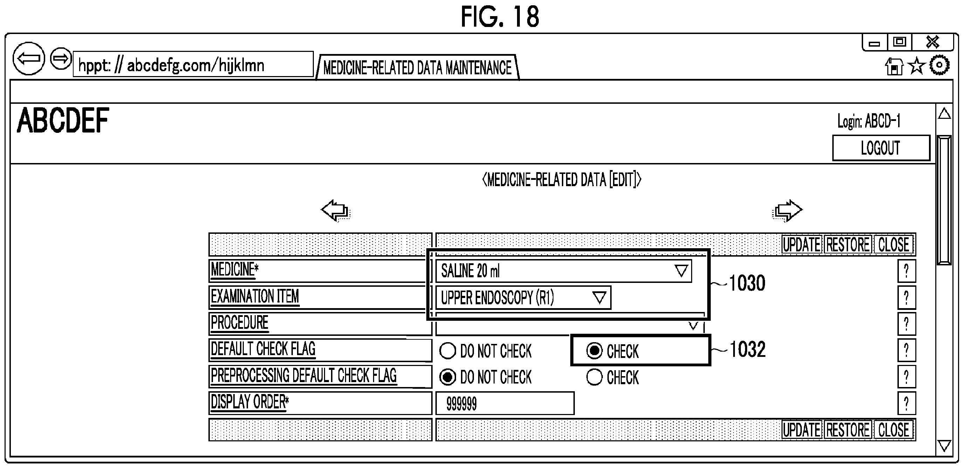

Fig. 17 is a diagram showing a scene in which the medicine is associated with the examination item. In a case where the user selects a medicine "saline 20 ml" and the examination item "upper endoscopy (R1)" on the screen shown in Fig. 17 (within a frame 1030), the database management unit 320A associates the medicine with the examination item. In a case where "check" is selected for the default check flag, the list editing unit 320G (list editing unit) sets the medicine "saline 20 ml" as the default check item (designated use article) of the examination item "upper endoscopy (R1)", but "do not check" is selected (within a frame 1031) in the example of Fig. 17. Similarly, Fig. 18 is a diagram showing a scene in which the medicine is associated with the examination item, but "check" is selected for the default check flag (in a frame 1032), and the list editing unit 320G sets the medicine "saline 20 ml" as a default check item (designated use medicine) of the examination item "upper endoscopy (Rl)". The frames in Figs. 17 and 18 are frames for description, and are not displayed on the actual screen.

### <Association of procedure with medicine and material>

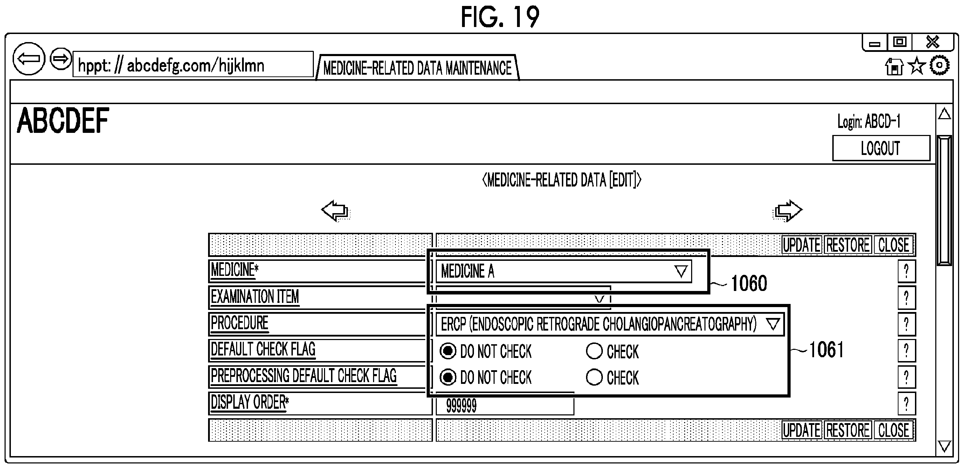

Fig. 19 is a diagram showing a scene in which the procedure is associated with the medicine. In a case where the user selects the medicine "medicine A" and the procedure "endoscopic retrograde cholangiopancreatography (ERCP)" on the screen shown in Fig. 19 (within frames 1060 and 1061 of Fig. 19), the database management unit 320A associates the procedure with the medicine. In a case where "check" is selected for the default check flag in the frame 1061, the list editing unit 320G (list editing unit) sets the medicine "medicine A" as the default check item (designated use article) of the procedure "ERCP", but "do not check" is selected (within the frame 1061) in the example of Fig. 19. Although Fig. 19 illustrates the association of the procedure with the medicine, the association of the procedure with the material (medical material) can be similarly processed. The frames 1060 and 1061 in Fig. 19 are frames for description, and are not displayed on the actual screen.

### <New creation of execution set>

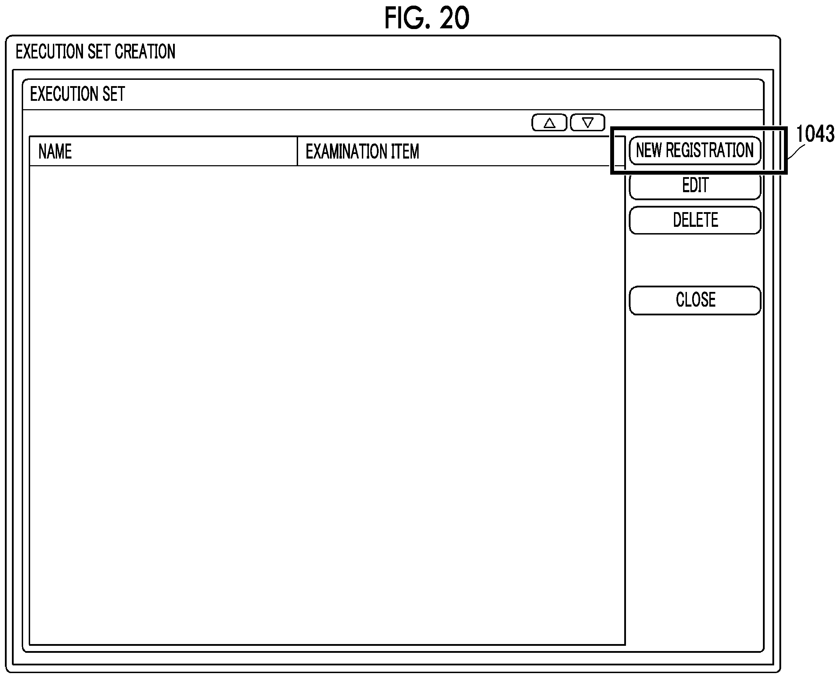

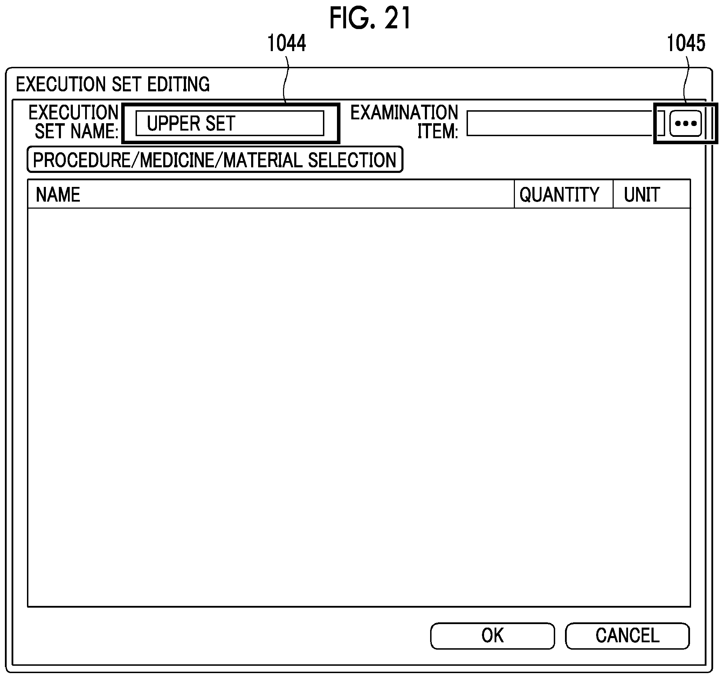

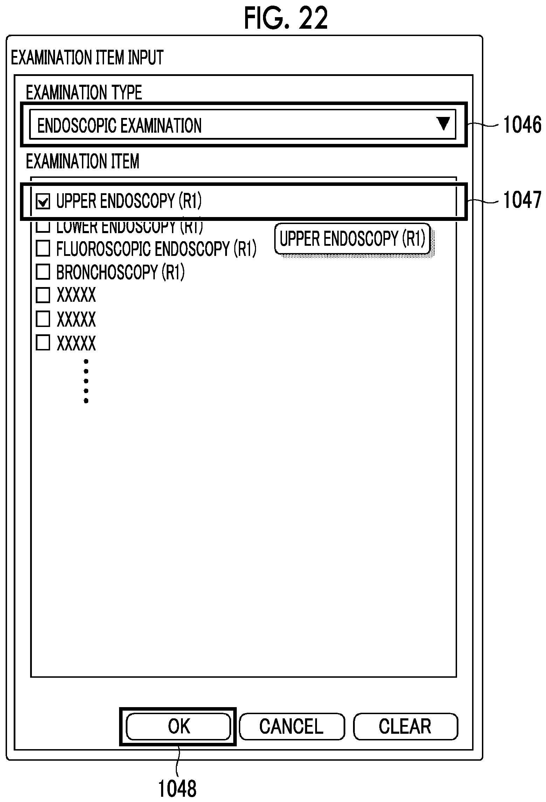

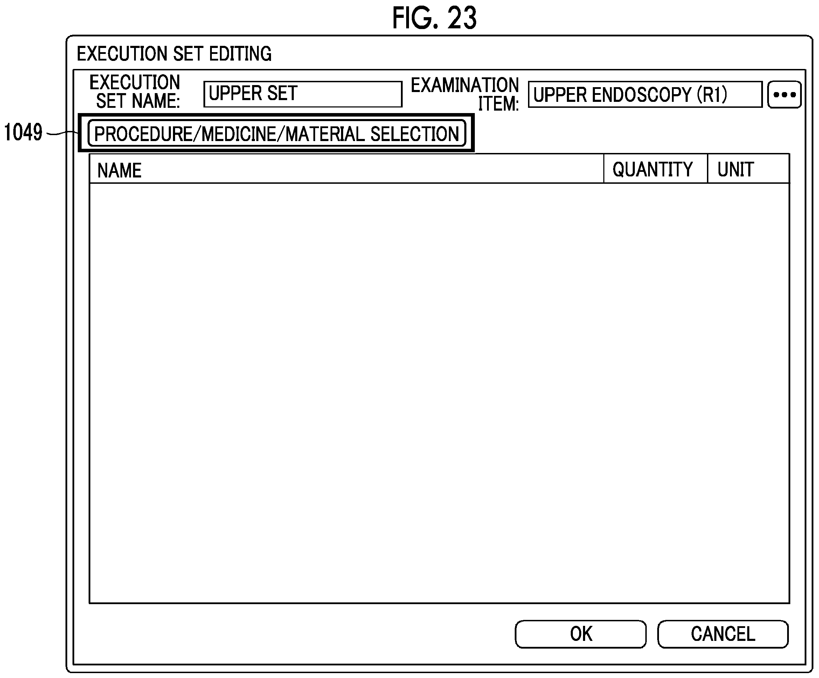

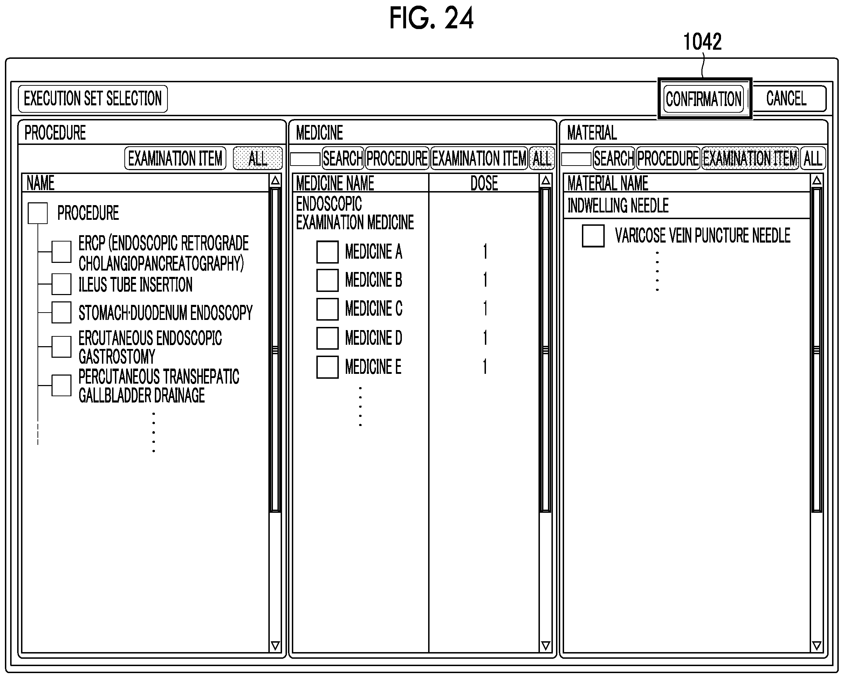

New creation of the execution set will be described. The following processing can be performed by the cloud server 320 (the information calculation unit 320E, the display controller 320F, the execution set editing unit 320H, and the like) according to the operation of the user via the input unit 110B of the client computer 110. In a case where "execution set creation" is designated on an execution report screen, an execution set list screen is displayed on the display unit 110A as shown in Fig. 20. In the example of Fig. 20, since even one execution set is not registered, a name of the execution set and the like are not displayed. In a case where a button 1043 of "new registration" is designated on this screen, the screen changes to an execution set editing screen as shown in Fig. 21. In Fig. 21, an execution set name to be registered (hereinafter, referred to as an "upper set") is input in a field of "execution set name" indicated by a frame 1044 at an upper left portion of the screen, and the examination item related to the execution set name is set by designation in a frame 1045 at an upper right portion of the screen. In the setting of the examination item, the screen changes to a screen shown in Fig. 22. On the screen of Fig. 22, the examination item associated with the execution set is selected by selecting an examination type associated with the execution set from a pull-down menu 1046 of "examination type" and checking a check box in a field of "examination item". In the example of Fig. 22, "upper endoscopy (R1)" indicated by a frame 1047 is selected. In a case where the examination type and the examination item are selected and an button 1048 of "OK" is designated, the screen changes to a screen for selecting the procedure, the medicine, and the material to be included in the execution set as shown in Fig. 23. On the screen of Fig. 23, in a case where a button 1049 of "procedure/medicine/material selection" is designated, selectable procedures, medicines, and materials are displayed as shown in Fig. 24, and the user checks check boxes for the procedures, medicines, and materials to be included in the execution set among the displayed procedures, medicines, and materials.

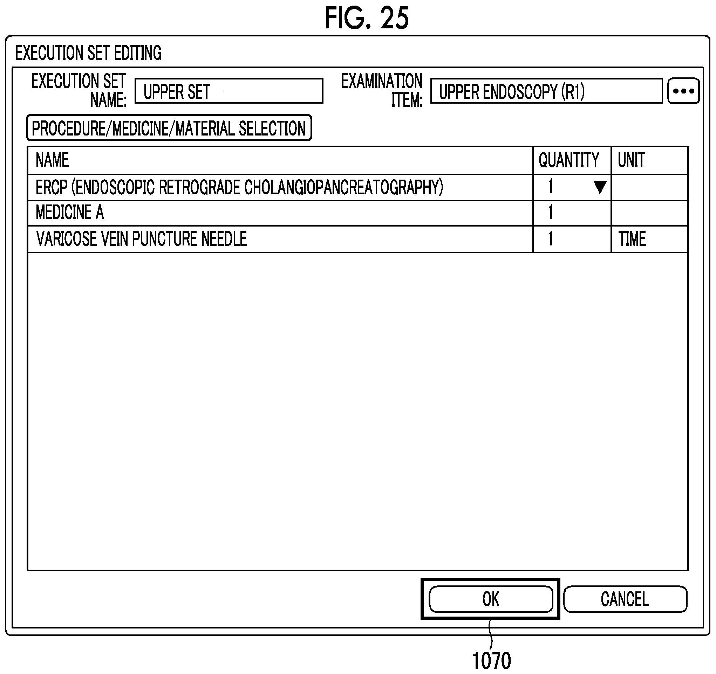

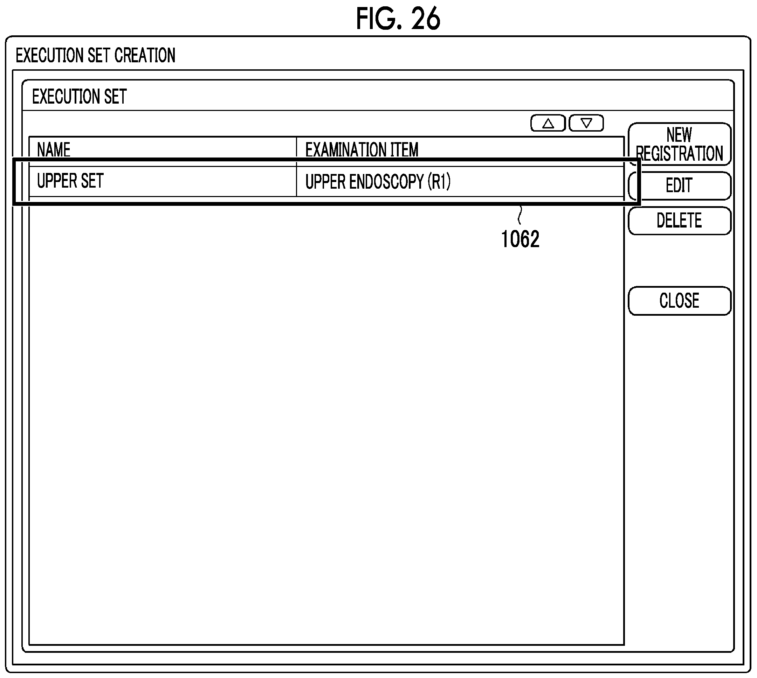

On a screen as shown in Fig. 24, it is preferable that the procedures, medicines, and materials are displayed in descending order of simultaneous execution rates for the examination item based on the update result of the examination database 322 using the database update unit 320C and the calculation result of the simultaneous execution rates (use rates and execution rates) using the information calculation unit 320E (are displayed in the order shown in the examples of Figs. 8 to 11, for example). Accordingly, the user can easily select an appropriate procedure, medicine, and material, and can easily register an optimal execution set. The simultaneous execution rates may be displayed by letters, numbers, symbols, and the like for each procedure, medicine, and material. On the screen of Fig. 24, a button 1042 of "confirm" is designated, the screen changes to a registration screen of the execution set as shown in Fig. 25, and the use quantity of the procedure, the medicine, and material may be changed. In a case where a button 1070 of "OK" is designated after the change is completed, the execution set editing unit 320H registers the execution set, and the execution set is displayed as shown in Fig. 26. In Fig. 26, a newly created and registered execution set is displayed in a frame 1062.

### <Editing of execution set>

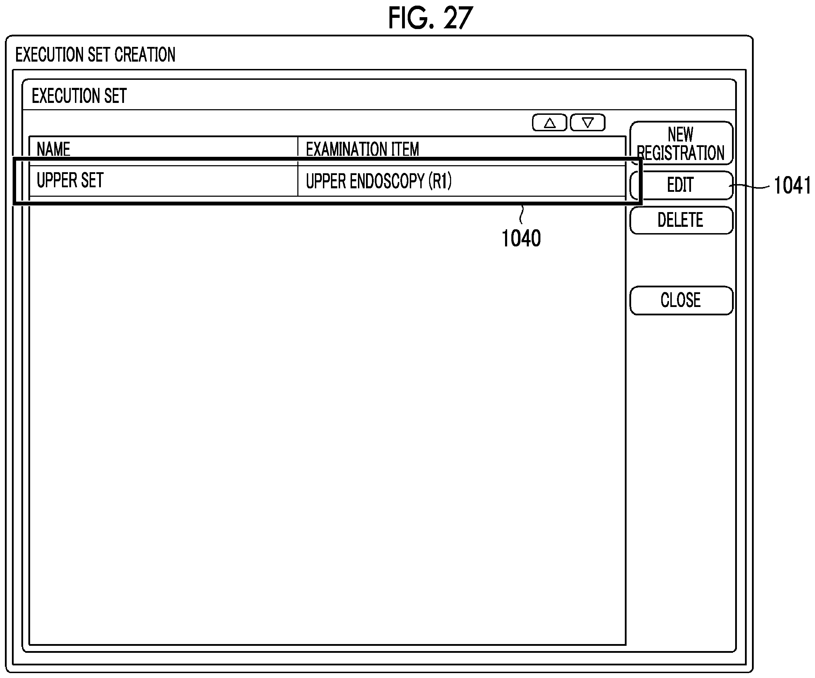

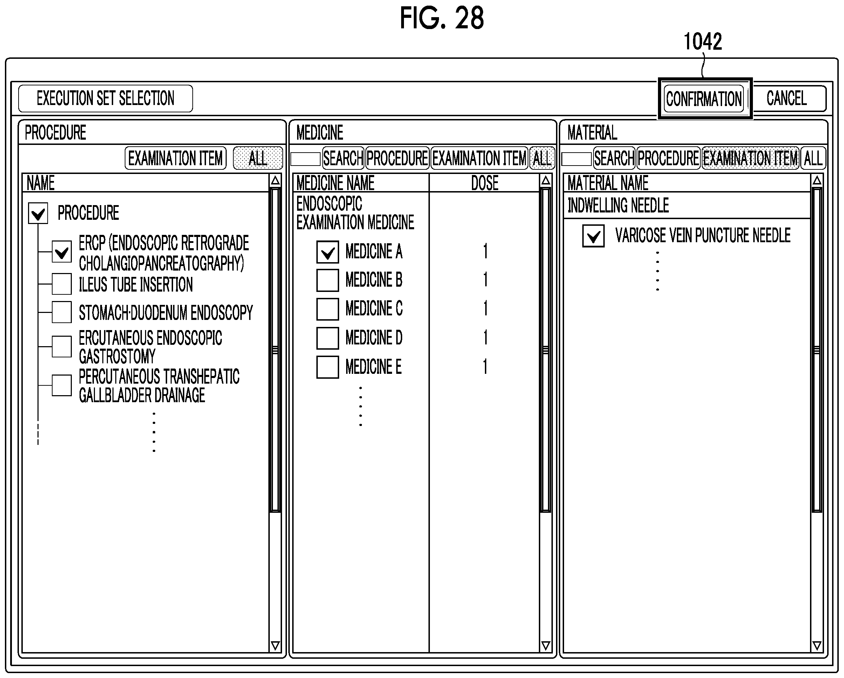

Fig. 27 is a diagram showing a scene (within a frame 1040) in which the execution set which has a name of "upper set" and is associated with the examination item "upper endoscopy (R1)" is registered and displayed on the display unit 110A. The user can instruct to edit the execution set by designating a button of "edit" with a device such as a mouse (not shown) of the input unit 110B. In a case where a button 1041 of "edit" is designated in the state of Fig. 27, the names of the procedure, medicine, and material name are displayed as shown in Fig. 28. Here, the check boxes are checked for the procedure, medicine, and material (medical material) registered in the selected execution set (here, "upper set" shown in Fig. 27). The user can instruct to exclude unnecessary procedure, medicine, and material or add necessary procedure, medicine, and material by checking necessary check boxes or unchecking the check boxes while confirming the check state of the check boxes. In a case where the button 1042 of "confirm" is designated, the editing is confirmed. The execution set editing unit 320H (execution set editing unit) edits the combination of the examination use article and procedure in the execution set according to the instruction of the user.

### <Support of input of examination information in case of execution report>

### <Extraction and display of input candidates based on examination item>

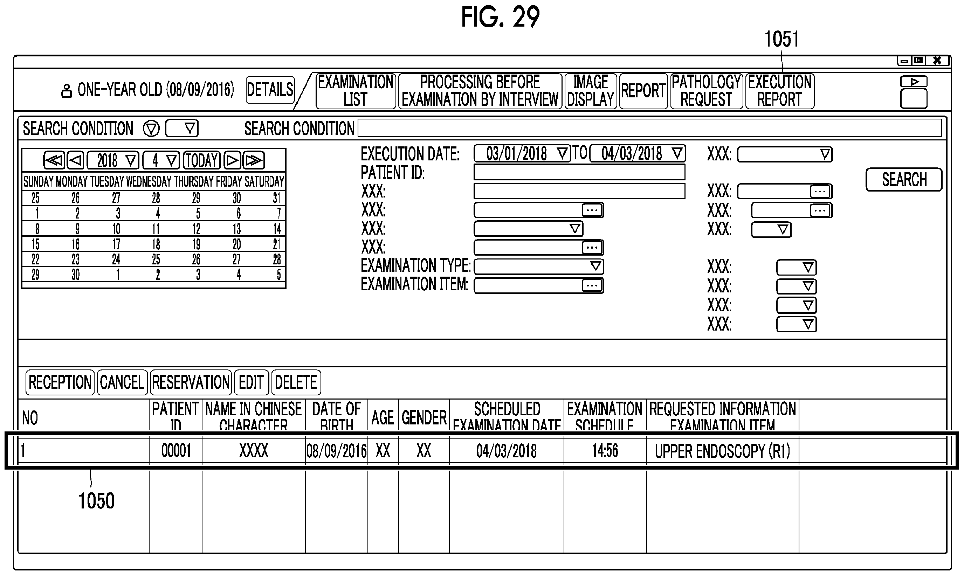

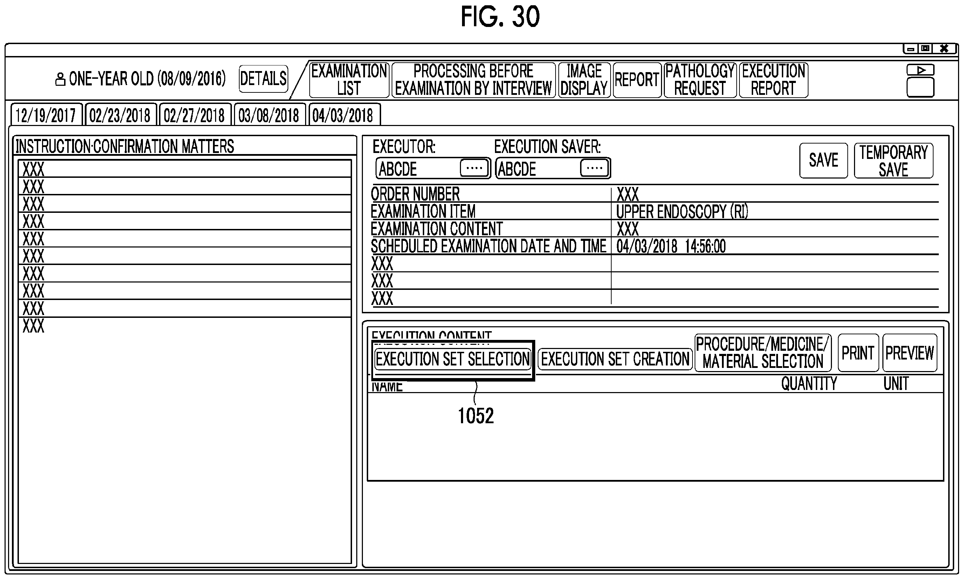

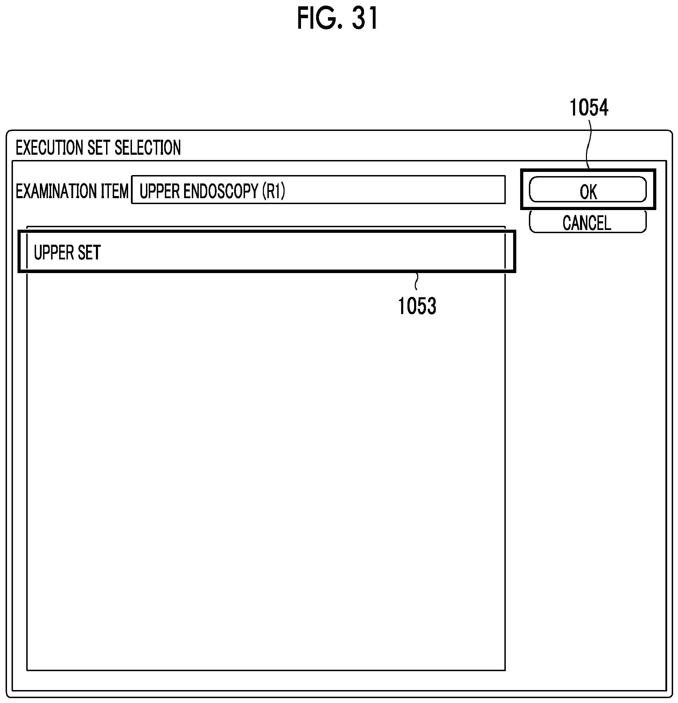

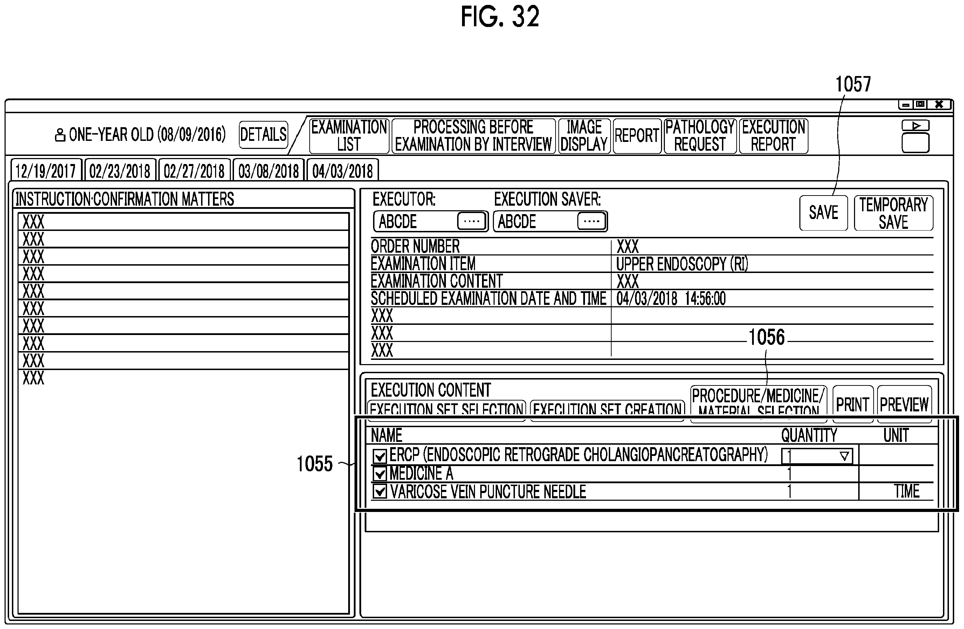

Fig. 29 is a diagram showing a scene in which examination information input is supported in reporting the execution after the examination is completed. Since a screen as shown in Fig. 29 is displayed on the display unit 110A of the client computer 110, the user selects the examination (indicated by a frame 1050) for reporting the execution via the input unit 110B, and designates a button 1051 of "execution report". Accordingly, information such as the examination item and the examination date and time is automatically input as shown in Fig. 30. In a case where the user designates a button 1052 of "execution set selection", the display controller 320F displays the execution set (here, only "upper set") related to the examination item (upper endoscopy (R1)) as shown in Fig. 31. The user selects a button 1053 of "upper set", and designates a button 1054 of "OK". Accordingly, the candidate extraction unit 320J (candidate extraction unit) extracts, as candidates for the examination execution procedure and candidates for the examination use article, the procedures, medicines, and materials registered in the list (default check items) for "upper set" which is the designated execution set and the examination item while referring to the examination database 322 based on the examination items. As shown in Fig. 32, the candidate extraction unit 320J displays the candidates in a region 1055 of "name" of the display screen of the display unit 110A (display device).

The candidates for the procedures, medicines, and materials displayed in Fig. 32 can be excluded from the input by unchecking the check boxes. The quantity can be correct via the pull-down menu of "quantity". In a case where a button 1056 of "procedure/medicine/material selection" is designated for the displayed candidates for the procedures, medicines, and materials, the same screen as that in Fig. 28 is displayed, and thus, the procedure, medicine, and material can be further selected and added.

In a case where the input of the procedure, medicine, or material used or executed in the examination is completed by the above processing, the user designates a button 1057 of "save". The instruction input unit 320K issues an instruction to register, as the examination information, at least a part of the displayed candidates (the result of the addition or the deletion of the displayed items) according to this operation. Accordingly, the examination information is transmitted to a payment system (reception), and is also transmitted to the cloud server 320. Thus, the recording in the examination database 322, the acquisition of the examination information, and the updating of the examination database 322 are performed as described above (examination database 322, database management unit 320A, examination information acquisition unit 320B, and database update unit 320C).

In the extraction of the candidates for the examination use articles, the procedures, medicines, and materials registered in the list and the execution set are extracted as the candidates while referring to the list (default check items) the execution set related to the examination item. In the endoscopic examination system 10 according to the first embodiment, since the use rate of the examination use article and the execution rate of the procedure are calculated and displayed as described above, the list of the designated use articles and the execution set can be appropriately edited. Thus, a situation of "medicines that are not used or not executed are registered in list or execution set" or a situation of "medicines to be used or executed are not registered in list or execution set" is reduced, and thus, the candidates extracted based on the list and the execution set reflect the use information and the execution information. Accordingly, time and effort to correct and input the candidates are less. Therefore, the endoscopic examination system 10 can efficiently support the input of the examination information based on an appropriate candidate.

### <Extraction and display of candidates based on procedure and extraction and display of candidates based on examination item and procedure>

Although it has been described in the above-described example that the candidates are extracted and displayed while referring to the examination database 322 based on the examination item, the candidate extraction unit 320J may extract and display the candidates for the examination use article while referring to the examination database 322 based on the procedure instead of the examination item. The candidate extraction unit 320J may extract and display the candidates for the examination use article while referring to the examination database 322 based on the examination item and the procedure. Since the examination items, procedures, medicines, and the like can be associated as shown in Figs. 15 to 19, it is possible to extract and display the candidates based on "procedure" or "examination item and procedure" while referring to the information on this association. It is possible to efficiently support the input of the examination information based on appropriate candidate by the extraction and display of the candidates similarly a case where the candidates based on the examination items are extracted and displayed.

Although the embodiments and other aspects of the present invention have been described above, the present invention is not limited to the above-described aspects, and various modifications can be made without departing from the spirit of the present invention. Explanation of References

1: procedure
2: procedure
3: procedure
4: procedure
10: endoscopic examination system
100: in-hospital system
110: client computer
110A: display unit
110B: input unit
110C: communication controller
112: endoscopic department database
120: in-hospital server
130: in-hospital LAN
140: router
300: cloud server section
310: router
320: cloud server
320A: database management unit
320B: examination information acquisition unit
320C: database update unit
320D: examination item selection unit
320E: information calculation unit
320F: display controller
320G: list editing unit
320H: execution set editing unit
3201: update condition setting unit
320J: candidate extraction unit
320K: instruction input unit
322: examination database
900: message
1000: rectangle
1001: arrow
1010: message
1020: frame
1021: frame
1022: frame
1030: frame
1031: frame
1032: frame
1040: frame
1041: button
1042: button
1043: button
1044: frame
1045: frame
1046: pull-down menu
1047: frame
1048: button
1049: button
1050: frame
1051: button
1052: button
1054: button
1055: region
1056: button
1057: button
1060: frame
1061: frame
1062: frame
1070: button
A: medicine
B: medicine
C: medicine
D: medicine
E: medicine
F: medicine
U: material

## Claims

1. An endoscopic examination information analysis apparatus comprising:
an examination database in which at least ones of examination use articles used in an examination using an endoscope or procedures executed in the examination are recorded in association with a plurality of examination items of the examination;
an examination information acquisition unit that acquires examination information which is generated whenever the examination is executed and indicates the examination use article in the examination and/or the procedure in the examination;
a database update unit that updates the examination database based on the examination information;
an examination item selection unit that selects an examination item from among the plurality of examination items recorded in the examination database;
an information calculation unit that calculates use information of the examination use article based on a use history of the examination use article, and calculates execution information of the procedure based on an execution history of the procedure for the selected examination item; and
a display controller that displays the calculated use information and execution information on a display device for each examination use article or procedure.

2. The endoscopic examination information analysis apparatus according to claim 1,
wherein the examination database records the use history for a designated use article which is the examination use article designated to be used in advance and the execution history for a designated execution procedure which is the procedure designated to be executed in advance, and the use history for a selected use article which is the examination use article to be used by selection and the execution history for a selected execution procedure which is the procedure designated to be executed by selection,
the database update unit updates the use history for the designated use article and the execution history for the designated execution procedure, and the use history for the selected use article and the execution history for the selected execution procedure, and
the display controller displays the use information for the designated use article and the execution information for the designated execution procedure for the selected examination item, and the use information for the selected use article and the execution information for the selected execution procedure for the selected examination item with discrimination from each other.

3. The endoscopic examination information analysis apparatus according to claim 2, further comprising:
a list editing unit that edits a list of the designated use article and the designated execution procedure.

4. The endoscopic examination information analysis apparatus according to claim 3,
wherein the display controller displays information prompting the list to be edited based on the use information and/or the execution information on the display device.

5. The endoscopic examination information analysis apparatus according to any one of claims 1 to 4,
wherein the display controller displays the use information for the examination use article included in an execution set which is information indicating a combination of the examination use article and the procedure and the execution information for the procedure included in the execution set with discrimination from the use information for the examination use article which is not included in the execution set and the execution information for the procedure which is not included in the execution set.

6. The endoscopic examination information analysis apparatus according to claim 5, further comprising:
an execution set editing unit that edits the combination of the examination use article and the procedure in the execution set.

7. The endoscopic examination information analysis apparatus according to claim 6,
wherein the display controller displays information prompting the combination to be edited based on the use information and the execution information on the display device.

8. The endoscopic examination information analysis apparatus according to any one of claims 1 to 7,
wherein the examination information acquisition unit acquires the examination information from a plurality of medical institutions, and
the information calculation unit calculates the use information and the execution information independently for each of the plurality of medical institutions.

9. The endoscopic examination information analysis apparatus according to any one of claims 1 to 7,
wherein the examination information acquisition unit acquires the examination information from a plurality of medical institutions, and
the information calculation unit calculates the use information and the execution information over two or more medical institutions of the plurality of medical institutions.

10. The endoscopic examination information analysis apparatus according to any one of claims 1 to 9, further comprising:
an update condition setting unit that sets an update condition of the examination database,
wherein the database update unit updates the examination database based on the set update condition.

11. The endoscopic examination information analysis apparatus according to any one of claims 1 to 10,
wherein the information calculation unit calculates, as the use information, a use rate expressed by a ratio of a total number of times of use for each medicine and medical material included in the examination use article to a total number of times of execution of the examination, and calculates, as the execution information, an execution rate expressed by a ratio of a total number of times of execution of the procedure to a total number of times of execution of the examination.

12. The endoscopic examination information analysis apparatus according to any one of claims 1 to 11,
wherein the examination use article includes at least one of a medicine or a medical material.

13. The endoscopic examination information analysis apparatus according to any one of claims 1 to 12,
wherein the display controller displays the calculated use information for each medicine and medical material on the display device.

14. An endoscopic examination information input support system comprising:
the endoscopic examination information analysis apparatus according to any one of claims 1 to 13;
the display device;
a candidate extraction unit that extracts candidates for the examination use article to be used in the examination while referring to the examination database, and displays the extracted candidates for the examination use article on the display device; and
an instruction input unit that inputs an instruction to register, as the examination information, at least a part of the displayed candidates,
wherein the candidate extraction unit extracts the candidates for the examination use article to be used in the examination while referring to the examination database based on at least one of the examination item selected from among the plurality of examination items or the selected procedure.

15. An endoscopic examination information analysis method comprising:
a recording step of recording at least ones of examination use articles used in an examination using an endoscope or procedures executed in the examination in association with a plurality of examination items of the examination in an examination database;
an examination information acquisition step of acquiring examination information which is generated whenever the examination is executed and indicates the examination use article in the examination and/or the procedure in the examination;
a database update step of updating the examination database based on the acquired examination information;
an examination item selection step of selecting an examination item from among the plurality of examination items recorded in the examination database;
an information calculation step of calculating use information of the examination use article based on a use history of the examination use article and calculating execution information of the procedure based on an execution history of the procedure for the selected examination item; and
a display control step of displaying the calculated use information and execution information on a display device for each examination use article or procedure.

16. The endoscopic examination information analysis method according to claim 15,
wherein, in the recording step, at least ones of medicines or medical materials are recorded as the examination use articles.

17. The endoscopic examination information analysis method according to claim 15 or 16,
wherein, in the display control step, the calculated use information is displayed on the display device for each medicine and medical material.
